# EUROPEAN PATENT SPECIFICATION

(11) **EP 1 981 960 B1**
(45) Date of publication and mention of the grant of the patent: **07.04.2010**
(21) Application number: 06825649.4
(22) Date of filing: 06.10.2006
(51) Int. Cl.: C11D 3/20, C11D 3/39, C11D 3/386, C07C 409/24, C12P 7/40

(54) **ENZYMATIC PRODUCTION OF PERACIDS FROM CARBOXYLIC ACID ESTER SUBSTRATES USING NON-HEME HALOPEROXIDASES**
ENZYMATISCHE HERSTELLUNG VON PERSÄUREN AUS CARBONSÄUREESTERSUBSTRATEN MITTELS NICHT-HÄM-HALOPEROXIDASEN
PRODUCTION ENZYMATIQUE DE PERACIDES A PARTIR DE SUBSTRATS D'ESTER D'ACIDE CARBOXYLIQUE AU MOYEN D'HALOPEROXYDASES NON HEME

(30) Priority: 06.10.2005 US 724106 P
(43) Date of publication of application: 22.10.2008
(73) Proprietor: E.I. DU PONT DE NEMOURS AND COMPANY, Wilmington, DE 19898 (US)
(72) Inventor: DICOSIMO, Robert, Chadds Ford, Pennsylvania 19317-9720 (US); PAYNE, Mark, S., Wilmington, Delaware 19808 (US); WAGNER, Lorraine, Newark, Delaware 19713 (US); GAVAGAN, John, E., Wilmington, Delaware 19810 (US)
(74) Representative: Webber, Philip Michael
(86) International application number: PCT/US2006/039395
(87) International publication number: WO 2007/044667

(56) References cited:
- EP-A2- 0 268 456
- EP-A2- 0 310 952
- EP-A2- 0 375 102
- WO-A-89/09813
- WO-A-94/18299
- WO-A-2006/119060
- WO-A2-96/06909
- US-A- 3 974 082
- US-A- 5 108 457
- US-A- 5 296 161
- US-B1- 6 703 540

### SEQUENCE LISTING

<110> E.I.duPont de Nemours and Company, Inc.
   DiCosimo, Robert
   Payne, Mark S.
   Wagner, Lorraine
   Gavagan, John E.
<120> ENZYMATIC PRODUCTION OF PERACIDS FROM CARBOXYLIC ACID ESTER SUBSTRATES USING NON-HEME HALOPEROXIDASES
<130> CL3090 PCT
<150> US 60/724,106
   <151> 2005-10-06
<160> 32
<170> PatentIn version 3.3
<210> 1
   <211> 27
   <212> DNA
   <213> artificial sequence
<220>
   <223> Primer
<400> 1
   gatctggtca tcgtcgccat gcatcac 27
<210>
   <211> 24
   <212> DNA
   <213> artificial sequence
<220>
   <223> Primer
<400> 2
   gccgacgact gggacgcgca gatg 24
<210> 3
   <211> 26
   <212> DNA
   <213> artificial sequence
<220>
   <223> Primer
<400> 3
   atgagctacg tcaccaccaa agatgg 26
<210> 4
   <211> 585
   <212> DNA
   <213> Pseudomonas putida 5B
<400> 4
   gccgacgact gggacgcgca gatgctgttc ttcctcgccc agggttaccg cgtggtcgcc 60
   cacgaccggc ggggccatgg ccgctccagc caggtatggg acggccacga catggaccac 120
   tatgccgacg acgtagccgc cgtggtcgcc cacctgggca cccagggcgc cgtgcatgtc 180
   ggccactcga ccggcggcgg cgaagtggtg cgctacatgg cccggcaccc tgaagacaag 240
   gtggccaagg ccgtgctgat cgcggccgtc ccccccttga tggtacaaac ccccggcaac 300
   cccggtggcc tgcccaagtc ggtattcgac ggtttccagg cccaggttgc cagcaaccgg 360
   gcgcagttct accgggatgt gccaacaggg ccgttctacg gctacaaccg cccaggggtc 420
   gaagccagcg aaggcatcat cggcaactgg tggcgccaag gcatgatcgg cagtgcaaag 480
   gcccattacg atggcatcgt ggcgttttcc cagaccgatt tcaccgagga cctgaagggc 540
   atccagcagc cggtgctggt gatgcatggc gacgatgacc agatc 585
<210> 5
   <211> 687
   <212> DNA
   <213> Pseudomonas putida 5B
<400> 5 <210> 6
   <211> 24
   <212> DNA
   <213> artificial sequence
<220>
   <223> Primer
<400> 6
   ggctacgtcg tcggcatagt ggtc 24
<210> 7
   <211> 27
   <212> DNA
   <213> artificial sequence
<220>
   <223> Primer
<400> 7
   ggtggaagtg gatcaccggt gcatcgc 27
<210> 8
   <211> 23
   <212> DNA
   <213> artificial sequence
<220>
   <223> Primer
<400> 8
   gcccattacg atggcatcgt ggc 23
<210> 9
   <211> 831
   <212> DNA
   <213> Pseudomonas putida 5B
<220>
   <221> CDS
   <222> (1)..(831)
<400> 9
   atg agc tat gta acc acg aag gac ggc gta cag att ttc tac aag gac      48
   Met Ser Tyr Val Thr Thr Lys Asp Gly Val Gln Ile Phe Tyr Lys Asp
   1               5                   10                  15
   tgg ggc ccg cgc gat gca ccg gtg atc cac ttc cac cac ggc tgg ccg      96
   Trp Gly Pro Arg Asp Ala Pro Val Ile His Phe His His Gly Trp Pro
               20                  25                  30
   ctc agt gcc gat gac tgg gac gcg cag atg ctg ttc ttc ctc gcc cag      144
   Leu Ser Ala Asp Asp Trp Asp Ala Gln Met Leu Phe Phe Leu Ala Gln
           35                  40                  45
   ggt tac cgc gtg gtc gcc cac gac cgg cgg ggc cat ggc cgc tcc agc      192
   Gly Tyr Arg Val Val Ala His Asp Arg Arg Gly His Gly Arg Ser Ser
       50                  55                  60
   cag gta tgg gac ggc cac gac atg gac cac tat gcc gac gac gta gcc      240
   Gln Val Trp Asp Gly His Asp Met Asp His Tyr Ala Asp Asp Val Ala
   65                  70                  75                  80
   gcc gtg gtc gcc cac ctg ggc acc cag ggc gcc gtg cat gtc ggc cac      288
   Ala Val Val Ala His Leu Gly Thr Gln Gly Ala Val His Val Gly His
                   85                  90                  95
   tcg acc ggc ggc ggc gaa gtg gtg cgc tac atg gcc cgg cac cct gaa      336
   Ser Thr Gly Gly Gly Glu Val Val Arg Tyr Met Ala Arg His Pro Glu
               100                 105                 110
   gac aag gtg gcc aag gcc gtg ctg atc gcg gcc gtc ccc ccc ttg atg      384
   Asp Lys Val Ala Lys Ala Val Leu Ile Ala Ala Val Pro Pro Leu Met
           115                 120                 125
   gta caa acc ccc ggc aac ccc ggt ggc ctg ccc aag tcg gta ttc gac      432
   Val Gln Thr Pro Gly Asn Pro Gly Gly Leu Pro Lys Ser Val Phe Asp
       130                 135                 140
   ggt ttc cag gcc cag gtt gcc agc aac cgg gcg cag ttc tac cgg gat      480
   Gly Phe Gln Ala Gln Val Ala Ser Asn Arg Ala Gln Phe Tyr Arg Asp
   145                 150                 155                 160
   gtg cca aca ggg ccg ttc tac ggc tac aac cgc cca ggg gtc gaa gcc      528
   Val Pro Thr Gly Pro Phe Tyr Gly Tyr Asn Arg Pro Gly Val Glu Ala
                   165                 170                 175
   agc gaa ggc atc atc ggc aac tgg tgg cgc caa ggc atg atc ggc agt      576
   Ser Glu Gly Ile Ile Gly Asn Trp Trp Arg Gln Gly Met Ile Gly Ser
               180                 185                 190
   gca aag gcc cat tac gat ggc atc gtg gcg ttt tcc cag acc gat ttc      624
   Ala Lys Ala His Tyr Asp Gly Ile Val Ala Phe Ser Gln Thr Asp Phe
           195                 200                 205
   acc gag gac ctg aag ggc atc cag cag ccg gtg ctg gtg atg cac ggc      672
   Thr Glu Asp Leu Lys Gly Ile Gln Gln Pro Val Leu Val Met His Gly
       210                 215                 220
   gac gat gac cag atc gtg cca tac gaa aat tcc ggg gta ctg tcg gcc      720
   Asp Asp Asp Gln Ile Val Pro Tyr Glu Asn Ser Gly Val Leu Ser Ala
   225                 230                 235                 240
   agg ctg ctg ccc aat ggc acg ctg aaa acc tac aaa ggc tac ccg cat      768
   Arg Leu Leu Pro Asn Gly Thr Leu Lys Thr Tyr Lys Gly Tyr Pro His
                   245                 250                 255
   ggc atg ccc acc acc cat gcc gat gtg atc aat gcg gat ttg ctg gcg      816
   Gly Met Pro Thr Thr His Ala Asp Val Ile Asn Ala Asp Leu Leu Ala
               260                 265                 270
   ttt atc cgt agt tga                                                  831
   Phe Ile Arg Ser
           275
<210> 10
   <211> 276
   <212> PRT
   <213> Pseudomonas putida 5B
<400> 10
   Met Ser Tyr Val Thr Thr Lys Asp Gly Val Gln Ile Phe Tyr Lys Asp
   1               5                   10                  15
   Trp Gly Pro Arg Asp Ala Pro Val Ile His Phe His His Gly Trp Pro
               20                  25                  30
   Leu Ser Ala Asp Asp Trp Asp Ala Gln Met Leu Phe Phe Leu Ala Gln
           35                  40                  45
   Gly Tyr Arg Val Val Ala His Asp Arg Arg Gly His Gly Arg Ser Ser
       50                  55                  60
   Gln Val Trp Asp Gly His Asp Met Asp His Tyr Ala Asp Asp Val Ala
   65                  70                  75                  80
   Ala Val Val Ala His Leu Gly Thr Gln Gly Ala Val His Val Gly His
                   85                  90                  95
   Ser Thr Gly Gly Gly Glu Val Val Arg Tyr Met Ala Arg His Pro Glu
               100                 105                 110
   Asp Lys Val Ala Lys Ala Val Leu Ile Ala Ala Val Pro Pro Leu Met
           115                 120                 125
   Val Gln Thr Pro Gly Asn Pro Gly Gly Leu Pro Lys Ser Val Phe Asp
       130                 135                 140
   Gly Phe Gln Ala Gln Val Ala Ser Asn Arg Ala Gln Phe Tyr Arg Asp
   145                 150                 155                 160
   Val Pro Thr Gly Pro Phe Tyr Gly Tyr Asn Arg Pro Gly Val Glu Ala
                   165                 170                 175
   Ser Glu Gly Ile Ile Gly Asn Trp Trp Arg Gln Gly Met Ile Gly Ser
   180 185 190
   Ala Lys Ala His Tyr Asp Gly Ile Val Ala Phe Ser Gln Thr Asp Phe
   195 200 205
   Thr Glu Asp Leu Lys Gly Ile Gln Gln Pro Val Leu Val Met His Gly
   210 215 220
   Asp Asp Asp Gln Ile Val Pro Tyr Glu Asn Ser Gly Val Leu Ser Ala
   225 230 235 240
   Arg Leu Leu Pro Asn Gly Thr Leu Lys Thr Tyr Lys Gly Tyr Pro His
   245 250 255
   Gly Met Pro Thr Thr His Ala Asp Val Ile Asn Ala Asp Leu Leu Ala
   260 265 270
   Phe Ile Arg Ser
   275
<210> 11
   <211> 33
   <212> DNA
   <213> artificial sequence
<220>
   <223> Primer
<400> 11
   gaattcatga gctatgtaac cacgaaggac ggc 33
<210> 12
   <211> 40
   <212> DNA
   <213> artificial sequence
<220>
   <223> Primer
<400> 12
   gcggccgctt aactacggat aaacgccagc aaatccgcat 40
<210> 13
   <211> 978
   <212> DNA
   <213> Agrobacterium tumefaciens C58 <220> <221> CDS <222> (1)..(978) <400> 13
<210> 14
   <211> 325
   <212> PRT
   <213> Agrobacterium tumefaciens C58
<400> 14
   Met Ala Ser Asp Thr Asp Arg Phe Phe Val Ser Arg Arg Ser Val Leu
   1               5                   10                  15
   Leu Gly Ser Leu Ala Leu Ala Ser Val Ser Val Pro Ala Leu Ala Phe
               20                  25                  30
   Ser His Thr Ser Ser Thr Asp Gly Ser Ser Gln Gly Asp Ile Asp Met
           35                  40                  45
   Gly Phe Val Thr Thr Lys Asp Gly Thr Glu Ile Phe Tyr Lys Asp Trp
       50                  55                  60
   Gly Pro Lys Asp Ala Gln Pro Ile Val Phe His His Gly Trp Pro Leu
   65                  70                  75                  80
   Ser Ser Asp Asp Trp Asp Ala Gln Met Leu Phe Phe Leu Ser Lys Gly
                   85                  90                  95
   Tyr Arg Val Val Ala His Asp Arg Arg Gly His Gly Arg Ser Ala Gln
               100                 105                 110
   Val Ala Asp Gly His Asp Met Asp His Tyr Ala Ala Asp Ala Phe Ala
           115                 120                 125
   Val Val Gln Ala Leu Asp Leu Lys Asn Ala Val His Ile Gly His Ser
       130                 135                 140
   Thr Gly Gly Gly Glu Val Ala Arg Tyr Val Ala Lys His Gly Gln Lys
   145                 150                 155                 160
   Ala Gly Arg Val Ala Lys Ala Val Leu Val Ser Ala Val Pro Pro Leu
                   165                 170                 175
   Met Val Lys Thr Thr Ala Asn Pro Glu Gly Leu Pro Ile Glu Val Phe
               180                 185                 190
   Asp Gly Phe Arg Ser Ala Leu Ala Ala Asn Arg Ala Gln Phe Phe Arg
           195                 200                 205
   Asp Val Pro Ala Gly Pro Phe Tyr Gly Phe Asn Arg Asp Gly Ala Lys
       210                 215                 220
   Val Gln Glu Gly Val Ile Gln Asn Trp Trp Arg Gln Gly Met Met Gly
   225                 230                 235                 240
   Gly Ala Lys Ala His Tyr Asp Gly Ile Lys Ala Phe Ser Glu Thr Asp
                   245                 250                 255
   Gln Thr Glu Asp Leu Thr Ala Ile Thr Val Pro Thr Leu Val Leu His
               260                 265                 270
   Gly Glu Asp Asp Gln Ile Val Pro Ile Ala Asp Ala Ala Arg Lys Ser
           275                 280                 285
   Ile Lys Leu Leu Lys Asn Gly Gln Ile Lys Thr Tyr Pro Gly Phe Ser
       290                 295                 300
   His Gly Met Leu Thr Val Asn Ala Asp Val Leu Asn Gln Asp Leu Leu
   305 310 315 320
   Ala Phe Ile Lys Gly
   325
<210> 15
   <211> 29
   <212> DNA
   <213> artificial sequence
<220>
   <223> Primer
<400> 15
   atgggcttcg taacaaccaa ggacggcac 29
<210> 16
   <211> 30
   <212> DNA
   <213> artificial sequence
<220>
   <223> Primer
<400> 16
   tcagcccttg atgaaggcta gcaggtcctg 30
<210> 17
   <211> 822
   <212> DNA
   <213> Agrobacterium tumefaciens C58
<220>
<221> CDS
   <222> (1)..(822)
<400> 17
   atg tcg cgt ttc acc acc act gac ggt acc cgc ctc ttt tat aag gac 48
   Met Ser Arg Phe Thr Thr Thr Asp Gly Thr Arg Leu Phe Tyr Lys Asp
   1 5 10 15
   tgg ggc gca ggg cag ccg atc ctc ttt gcc cac ggg tgg ccg ctg tca 96
   Trp Gly Ala Gly Gln Pro Ile Leu Phe Ala His Gly Trp Pro Leu Ser
   20 25 30
   tcc gat gca tgg gac cag cag atg ctg ttc ttc agc cag aac ggc ttc 144
   Ser Asp Ala Trp Asp Gln Gln Met Leu Phe Phe Ser Gln Asn Gly Phe
   35 40 45
   cgc gtc atc gcc cac gat cgc cgc agc cat ggc cgc tcg gat cag aca        192
   Arg Val Ile Ala His Asp Arg Arg Ser His Gly Arg Ser Asp Gln Thr
       50                  55                  60
   ttt gac ggc aac aac atg gac cag tat gcc gac gat ctc gcc gaa ctg        240
   Phe Asp Gly Asn Asn Met Asp Gln Tyr Ala Asp Asp Leu Ala Glu Leu
   65                  70                  75                  80
   atc aat gca ctc gac ctg tcg gac ctg atc ttg gtt ggc cat tcg acc        288
   Ile Asn Ala Leu Asp Leu Ser Asp Leu Ile Leu Val Gly His Ser Thr
                   85                  90                  95
   ggt ggc ggc gag gtc tcg cat tat gtc ggc cgt cat ggc acg gct cgt        336
   Gly Gly Gly Glu Val Ser His Tyr Val Gly Arg His Gly Thr Ala Arg
               100                 105                 110
   gtt gcc aag gtg gtt ctg gtg ggt gcg gtg ccg ccg gtg atg ctg aag        384
   Val Ala Lys Val Val Leu Val Gly Ala Val Pro Pro Val Met Leu Lys
           115                 120                 125
   acc gtt gac aac ccg aac ggt acg ccg atg gag gta ttc gac agt atc        432
   Thr Val Asp Asn Pro Asn Gly Thr Pro Met Glu Val Phe Asp Ser Ile
       130                 135                 140
   cgc gaa aac acc gcg aag aac cgt tcg cag ttt ttc ttc gat ctg acg        480
   Arg Glu Asn Thr Ala Lys Asn Arg Ser Gln Phe Phe Phe Asp Leu Thr
   145                 150                 155                 160
   gtg cct ttc tac ggc ttt aac cgg gaa ggt gtc gcg acc aat gac ggc        528
   Val Pro Phe Tyr Gly Phe Asn Arg Glu Gly Val Ala Thr Asn Asp Gly
                   165                 170                 175
   atg cgg gaa aac ttc cgc cgc ata gga ctg caa ggc ggc gtc aag ggc        576
   Met Arg Glu Asn Phe Arg Arg Ile Gly Leu Gln Gly Gly Val Lys Gly
               180                 185                 190
   cag tac gat tgc atc cgg gag ttc tct gag gtc gat tac acc cgt gat        624
   Gln Tyr Asp Cys Ile Arg Glu Phe Ser Glu Val Asp Tyr Thr Arg Asp
           195                 200                 205
   ctg aag agt att gac cgc ccg acg ctg atc atc cat ggc gat gac gac        672
   Leu Lys Ser Ile Asp Arg Pro Thr Leu Ile Ile His Gly Asp Asp Asp
       210                 215                 220
   cag atc gtt ccg atc gat gca tct gcc cat cgg gct gcg gac ctc gtg        720
   Gln Ile Val Pro Ile Asp Ala Ser Ala His Arg Ala Ala Asp Leu Val
   225                 230                 235                 240
   gcc gat gcc acg ctg aag atc tat tcg ggc ggc tcc cac gga ctg gca        768
   Ala Asp Ala Thr Leu Lys Ile Tyr Ser Gly Gly Ser His Gly Leu Ala
                   245                 250                 255
   gag act gag gct gat cgt ttc aac gcc gac gtg ctc gcc ttt atc cac        816
   Glu Thr Glu Ala Asp Arg Phe Asn Ala Asp Val Leu Ala Phe Ile His
               260                 265                 270
   ggc tga                                                                822
   Gly
<210> 18
   <211> 273
   <212> PRT
   <213> Agrobacterium tumefaciens C58
<400> 18
   Met Ser Arg Phe Thr Thr Thr Asp Gly Thr Arg Leu Phe Tyr Lys Asp
   1               5                   10                  15
   Trp Gly Ala Gly Gln Pro Ile Leu Phe Ala His Gly Trp Pro Leu Ser
               20                  25                  30
   Ser Asp Ala Trp Asp Gln Gln Met Leu Phe Phe Ser Gln Asn Gly Phe
           35                  40                  45
   Arg Val Ile Ala His Asp Arg Arg Ser His Gly Arg Ser Asp Gln Thr
       50                  55                  60
   Phe Asp Gly Asn Asn Met Asp Gln Tyr Ala Asp Asp Leu Ala Glu Leu
   65                  70                  75                  80
   Ile Asn Ala Leu Asp Leu Ser Asp Leu Ile Leu Val Gly His Ser Thr
                   85                  90                  95
   Gly Gly Gly Glu Val Ser His Tyr Val Gly Arg His Gly Thr Ala Arg
               100                 105                 110
   Val Ala Lys Val Val Leu Val Gly Ala Val Pro Pro Val Met Leu Lys
           115                 120                 125
   Thr Val Asp Asn Pro Asn Gly Thr Pro Met Glu Val Phe Asp Ser Ile
       130                 135                 140
   Arg Glu Asn Thr Ala Lys Asn Arg Ser Gln Phe Phe Phe Asp Leu Thr
   145                 150                 155                 160
   Val Pro Phe Tyr Gly Phe Asn Arg Glu Gly Val Ala Thr Asn Asp Gly
                   165                 170                 175
   Met Arg Glu Asn Phe Arg Arg Ile Gly Leu Gln Gly Gly Val Lys Gly
               180                 185                 190
   Gln Tyr Asp Cys Ile Arg Glu Phe Ser Glu Val Asp Tyr Thr Arg Asp
   195 200 205
   Leu Lys Ser Ile Asp Arg Pro Thr Leu Ile Ile His Gly Asp Asp Asp
   210 215 220
   Gln Ile Val Pro Ile Asp Ala Ser Ala His Arg Ala Ala Asp Leu Val
   225 230 235 240
   Ala Asp Ala Thr Leu Lys Ile Tyr Ser Gly Gly Ser His Gly Leu Ala
   245 250 255
   Glu Thr Glu Ala Asp Arg Phe Asn Ala Asp Val Leu Ala Phe Ile His
   260 265 270
   Gly
<210> 19
   <211> 30
   <212> DNA
   <213> artificial sequence
<220>
   <223> Primer
<400> 19
   atgtcgcgtt tcaccaccac tgacggtacc 30
<210> 20
   <211> 28
   <212> DNA
   <213> artificial sequence
<220>
   <223> Primer
<400> 20
   tcagccgtgg ataaaggcga gcacgtcg 28
<210> 21
   <211> 828
   <212> DNA
   <213> Agrobacterium tumefaciens C58
<220>
   <221> CDS
   <222> (1)..(828)
<400> 21
   atg aca acg atc acc acc aga gac gga acg cac atc ttc tac aag gac        48
   Met Thr Thr Ile Thr Thr Arg Asp Gly Thr His Ile Phe Tyr Lys Asp
   1               5                   10                  15
   tgg ggt ccg aaa gac ggc cag ccc atc atc ttc tcc cat ggc tgg ccg        96
   Trp Gly Pro Lys Asp Gly Gln Pro Ile Ile Phe Ser His Gly Trp Pro
               20                  25                  30
   ctc agc gcc gac acc tgg gat gcg cag atg gtg ttc ttc gcc aat aac       144
   Leu Ser Ala Asp Thr Trp Asp Ala Gln Met Val Phe Phe Ala Asn Asn
           35                  40                  45
   ggt ttc cgc acc atc gcc cat gac cgc cgc agc cac ggc cgc tcg gat       192
   Gly Phe Arg Thr Ile Ala His Asp Arg Arg Ser His Gly Arg Ser Asp
       50                  55                  60
   cag gtc tgg cac aac aat acc atg gat caa tat gcc gac gat ctc gcc       240
   Gln Val Trp His Asn Asn Thr Met Asp Gln Tyr Ala Asp Asp Leu Ala
   65                  70                  75                  80
   gag ttg atc gaa cag ctc gat ctc cac gac atc att cta gtc ggt cat       288
   Glu Leu Ile Glu Gln Leu Asp Leu His Asp Ile Ile Leu Val Gly His
                   85                  90                  95
   tcc acc ggc ggc ggc gag gtg acc cgt tat atc ggc cgc cac ggc acg       336
   Ser Thr Gly Gly Gly Glu Val Thr Arg Tyr Ile Gly Arg His Gly Thr
               100                 105                 110
   gcc cgc gtc gcg aag atc gcc ctc atc ggc gcg gtg ccg ccg ctg atg       384
   Ala Arg Val Ala Lys Ile Ala Leu Ile Gly Ala Val Pro Pro Leu Met
           115                 120                 125
   ctg aag acg gaa agc aat acc atc ggc atg ccg atc gat gtc ttc gat       432
   Leu Lys Thr Glu Ser Asn Thr Ile Gly Met Pro Ile Asp Val Phe Asp
       130                 135                 140
   ggc atc cgc aag agc acc tat gat aat cgc agc cag ttc ttc aag gac       480
   Gly Ile Arg Lys Ser Thr Tyr Asp Asn Arg Ser Gln Phe Phe Lys Asp
   145                 150                 155                 160
   ctg acc att ccc ttc tac ggc tac aac cgc gaa ggc gcc gtg att tcg       528
   Leu Thr Ile Pro Phe Tyr Gly Tyr Asn Arg Glu Gly Ala Val Ile Ser
                   165                 170                 175
   gaa ggc att cgc gag gaa ttc tgg cgg cag ggc atg atg ggc ggc ctc       576
   Glu Gly Ile Arg Glu Glu Phe Trp Arg Gln Gly Met Met Gly Gly Leu
               180                 185                 190
   aag ggc cag ctc gac agt atc cgc gcc ttc tcg gaa agc gat ttc cac       624
   Lys Gly Gln Leu Asp Ser Ile Arg Ala Phe Ser Glu Ser Asp Phe His
           195                 200                 205
   cag gac ctg aag gcc ttc gac aaa cca acg ctg gtg ctg cat ggc gat       672
   Gln Asp Leu Lys Ala Phe Asp Lys Pro Thr Leu Val Leu His Gly Asp
       210                 215                 220
   gac gac cag atc gtg ccg gtc ggc gct tca gca ctt tcc acg gtc aag      720
   Asp Asp Gln Ile Val Pro Val Gly Ala Ser Ala Leu Ser Thr Val Lys
   225                 230                 235                 240
   atc gtc aaa cat gcg acc ctc aaa atc tac gag ggc gcc gac cat ggc      768
   Ile Val Lys His Ala Thr Leu Lys Ile Tyr Glu Gly Ala Asp His Gly
                   245                 250                 255
   ctg acg cag acc cat cag gac cgg ttc aac gcc gac ctc ctc acc ttc      816
   Leu Thr Gln Thr His Gln Asp Arg Phe Asn Ala Asp Leu Leu Thr Phe
               260                 265                 270
   atc aac gcc tga                                                      828
   Ile Asn Ala
           275
<210> 22
   <211> 275
   <212> PRT
   <213> Agrobacterium tumefaciens C58
<400> 22.
   Met Thr Thr Ile Thr Thr Arg Asp Gly Thr His Ile Phe Tyr Lys Asp
   1               5                   10                  15
   Trp Gly Pro Lys Asp Gly Gln Pro Ile Ile Phe Ser His Gly Trp Pro
               20                  25                  30
   Leu Ser Ala Asp Thr Trp Asp Ala Gln Met Val Phe Phe Ala Asn Asn
           35                  40                  45
   Gly Phe Arg Thr Ile Ala His Asp Arg Arg Ser His Gly Arg Ser Asp
       50                  55                  60
   Gln Val Trp His Asn Asn Thr Met Asp Gln Tyr Ala Asp Asp Leu Ala
   65                  70                  75                  80
   Glu Leu Ile Glu Gln Leu Asp Leu His Asp Ile Ile Leu Val Gly His
                   85                  90                  95
   Ser Thr Gly Gly Gly Glu Val Thr Arg Tyr Ile Gly Arg His Gly Thr
               100                 105                 110
   Ala Arg Val Ala Lys Ile Ala Leu Ile Gly Ala Val Pro Pro Leu Met
           115                 120                 125
   Leu Lys Thr Glu Ser Asn Thr Ile Gly Met Pro Ile Asp Val Phe Asp
   130 135 140
   Gly Ile Arg Lys Ser Thr Tyr Asp Asn Arg Ser Gln Phe Phe Lys Asp
   145 150 155 160
   Leu Thr Ile Pro Phe Tyr Gly Tyr Asn Arg Glu Gly Ala Val Ile Ser
   165 170 175
   Glu Gly Ile Arg Glu Glu Phe Trp Arg Gln Gly Met Met Gly Gly Leu
   180 185 190
   Lys Gly Gln Leu Asp Ser Ile Arg Ala Phe Ser Glu Ser Asp Phe His
   195 200 205
   Gln Asp Leu Lys Ala Phe Asp Lys Pro Thr Leu Val Leu His Gly Asp
   210 215 220
   Asp Asp Gln Ile Val Pro Val Gly Ala Ser Ala Leu Ser Thr Val Lys
   225 230 235 240
   Ile Val Lys His Ala Thr Leu Lys Ile Tyr Glu Gly Ala Asp His Gly
   245 250 255
   Leu Thr Gln Thr His Gln Asp Arg Phe Asn Ala Asp Leu Leu Thr Phe
   260 265 270
   Ile Asn Ala
   275
<210> 23
   <211> 30
   <212> DNA
   <213> artificial sequence
<220>
   <223> Primer
<400> 23
   atgacaacga tcaccaccag agacggaacg 30
<210> 24
   <211> 28
   <212> DNA
   <213> artificial sequence
<220>
   <223> Primer
<400> 24
   tcaggcgttg atgaaggtga ggaggtcg 28
<210> 25
   <211> 31
   <212> DNA
   <213> artificial sequence
<229>
   <223> Primer
<400> 25
   caattgatga caacgatcac caccagagac g 31
<210> 26
   <211> 43
   <212> DNA
   <213> artificial sequence
<220>
   <223> Primer
<400> 26
   caattgcggc cgcttaggcg ttgatgaagg tgaggaggtc ggc 43
<210> 27
   <211> 837
   <212> DNA
   <213> Agrobacterium tumefaciens C58
<220>
   <221> CDS
   <222> (1)..(837)
<400> 27
   atg ggc ttt gtt aag acg aca gac gga acc gaa atc ttt tac aag gac      48
   Met Gly Phe Val Lys Thr Thr Asp Gly Thr Glu Ile Phe Tyr Lys Asp
   1               5                   10                  15
   tgg ggc ccg aag gat gcc cag ccg atc atg ttc cac cac ggc tgg ccg      96
   Trp Gly Pro Lys Asp Ala Gln Pro Ile Met Phe His His Gly Trp Pro
               20                  25                  30
   ctc tcg tcc gat gat tgg gat gcg cag atg ctg ttt ttc ctc tct aaa     144
   Leu Ser Ser Asp Asp Trp Asp Ala Gln Met Leu Phe Phe Leu Ser Lys
           35                  40                  45
   ggt ttt cgt gtt gtc gcc cat gac cgc cgt ggt cac ggc cgc tcc gcc     192
   Gly Phe Arg Val Val Ala His Asp Arg Arg Gly His Gly Arg Ser Ala
       50                  55                  60
   cag gtt gcc gat ggt cac gat atg gac cac tac gcc gcc gac gcc ttc      240
   Gln Val Ala Asp Gly His Asp Met Asp His Tyr Ala Ala Asp Ala Phe
   65                  70                  75                  80
   gcc gtc gtt gag gcg ctc gat ctg aag aat gtc gtc cat atc ggc cat      288
   Ala Val Val Glu Ala Leu Asp Leu Lys Asn Val Val His Ile Gly His
                   85                  90                  95
   tcc acc ggt ggc ggc gaa gtc gcc cgt tat gtc gcc aag cat ggc gaa      336
   Ser Thr Gly Gly Gly Glu Val Ala Arg Tyr Val Ala Lys His Gly Glu
               100                 105                 110
   ccc gcc ggc cgc gtt gcc aag gcg gtt ctc gtt tcc gcg gta ccg ccg      384
   Pro Ala Gly Arg Val Ala Lys Ala Val Leu Val Ser Ala Val Pro Pro
           115                 120                 125
   ctg atg ctc aag acc gag gcc aat ccg gaa ggc ctg ccg atg gaa gtc      432
   Leu Met Leu Lys Thr Glu Ala Asn Pro Glu Gly Leu Pro Met Glu Val
       130                 135                 140
   ttt gac ggc ttc cgc tca gcc ctt gcc gca aac cga gcc cag ttc ttc      480
   Phe Asp Gly Phe Arg Ser Ala Leu Ala Ala Asn Arg Ala Gln Phe Phe
   145                 150                 155                 160
   cgc gac gtg ccg tcc ggc ccg ttc tac ggc ttc aac cgc gat ggc gcg      528
   Arg Asp Val Pro Ser Gly Pro Phe Tyr Gly Phe Asn Arg Asp Gly Ala
                   165                 170                 175
   tcc gtg cag gaa ggc gtg atc cag aac tgg tgg cgt cag ggc atg atg      576
   Ser Val Gln Glu Gly Val Ile Gln Asn Trp Trp Arg Gln Gly Met Met
               180                 185                 190
   ggc ggc gcg aag gct cat tat gac ggc gtc aag gcc ttc tcc gaa acc      624
   Gly Gly Ala Lys Ala His Tyr Asp Gly Val Lys Ala Phe Ser Glu Thr
           195                 200                 205
   gac cag act gaa gac ctg aag aat atc agc gtt cca acg ctc gtt ctc      672
   Asp Gln Thr Glu Asp Leu Lys Asn Ile Ser Val Pro Thr Leu Val Leu
       210                 215                 220
   cac ggt gaa gac gac cag atc gtt ccg att gcc gac tcg gct cac aaa      720
   His Gly Glu Asp Asp Gln Ile Val Pro Ile Ala Asp Ser Ala His Lys
   225                 230                 235                 240
   tcg gtc aag ctg ctg aag aac ggc acg ctg aag act tat ccc ggg ttc      768
   Ser Val Lys Leu Leu Lys Asn Gly Thr Leu Lys Thr Tyr Pro Gly Phe
                   245                 250                 255
   tcg cac ggc atg ctg aca gtc aat gcc gat gtg ctg aat gcg gat ctg      816
   Ser His Gly Met Leu Thr Val Asn Ala Asp Val Leu Asn Ala Asp Leu
               260                 265                 270
   ctg gcc ttc atc aag ggc tga                                          837
   Leu Ala Phe Ile Lys Gly
   275
<210> 28
   <211> 278
   <212> PRT
   <213> Agrobacterium tumefaciens C58
<400> 28
   Met Gly Phe Val Lys Thr Thr Asp Gly Thr Glu Ile Phe Tyr Lys Asp
   1               5                   10                  15
   Trp Gly Pro Lys Asp Ala Gln Pro Ile Met Phe His His Gly Trp Pro
               20                  25                  30
   Leu Ser Ser Asp Asp Trp Asp Ala Gln Met Leu Phe Phe Leu Ser Lys
           35                  40                  45
   Gly Phe Arg Val Val Ala His Asp Arg Arg Gly His Gly Arg Ser Ala
       50                  55                  60
   Gln Val Ala Asp Gly His Asp Met Asp His Tyr Ala Ala Asp Ala Phe
   65                  70                  75                  80
   Ala Val Val Glu Ala Leu Asp Leu Lys Asn Val Val His Ile Gly His
                   85                  90                  95
   Ser Thr Gly Gly Gly Glu Val Ala Arg Tyr Val Ala Lys His Gly Glu
               100                 105                 110
   Pro Ala Gly Arg Val Ala Lys Ala Val Leu Val Ser Ala Val Pro Pro
           115                 120                 125
   Leu Met Leu Lys Thr Glu Ala Asn Pro Glu Gly Leu Pro Met Glu Val
       130                 135                 140
   Phe Asp Gly Phe Arg Ser Ala Leu Ala Ala Asn Arg Ala Gln Phe Phe
   145                 150                 155                 160
   Arg Asp Val Pro Ser Gly Pro Phe Tyr Gly Phe Asn Arg Asp Gly Ala
                   165                 170                 175
   Ser Val Gln Glu Gly Val Ile Gln Asn Trp Trp Arg Gln Gly Met Met
               180                 185                 190
   Gly Gly Ala Lys Ala His Tyr Asp Gly Val Lys Ala Phe Ser Glu Thr
   195 200 205
   Asp Gln Thr Glu Asp Leu Lys Asn Ile Ser Val Pro Thr Leu Val Leu
   210 215 220
   His Gly Glu Asp Asp Gln Ile Val Pro Ile Ala Asp Ser Ala His Lys
   225 230 235 240
   Ser Val Lys Leu Leu Lys Asn Gly Thr Leu Lys Thr Tyr Pro Gly Phe
   245 250 255
   Ser His Gly Met Leu Thr Val Asn Ala Asp Val Leu Asn Ala Asp Leu
   260 265 270
   Leu Ala Phe Ile Lys Gly
   275
<210> 29
   <211> 30
   <212> DNA
   <213> artificial sequence
<220>
   <223> Primer
<400> 29
   atgggctttg ttaagacgac agacggaacc 30
<210> 30
   <211> 28
   <212> DNA
   <213> artificial sequence
<220>
   <223> Primer
<400> 30
   tcagcccttg atgaaggcca gcagatcc 28
<210> 31
   <211> 33
   <212> DNA
   <213> artificial sequence
<220>
   <223> Primer
<400> 31
   atgaattcat gggctttgtt aagacgacag acg 33
<210> 32
   <211> 37
   <212> DNA
   <213> artificial sequence
<220>
   <223> Primer
<400> 32
   atgcggccgc tcagcccttg atgaaggcca gcagatc 37

## Description

### FIELD OF THE INVENTION

This invention relates to the field of peracid biosynthesis and *in situ* enzyme catalysis. Specifically, non-heme haloperoxidases having perhydrolysis activity were used to produce peracids from carboxylic acid ester substrates.

### BACKGROUND OF THE INVENTION

Peracid compositions have been reported to be effective antimicrobial agents. Methods to clean, disinfect, and/or sanitize hard surfaces, meat products, living plant tissues, and medical devices against undesirable microbial growth have been described (US 6,545,047, US 6,183,807, US 6,518,307, US 20030026846, and US 5,683,724). Peracids have also been reported to be useful in preparing bleaching compositions for laundry detergent applications (US 3.974.082. US 5,296,161, and US 5,364,554). WO96/06909 discloses enzymatic, active oxygen-releasing mixtures which may be used as oxidising agents for preparing chemical compounds and in bleaching, washing, cleaning and disinfecting. The mixtures contain an oxidoreductase, a peroxide source, and an aqueous solution of an organic acid or its salt.

Peracids can be prepared by the chemical reaction of a carboxylic acid and hydrogen peroxide (see Organic Peroxides, Daniel Swern, ed., Vol. 1, pp 313-516; Wiley Interscience, New York). A strong inorganic acid, such as concentrated sulfuric acid, usually catalyzes the reaction. The reaction of hydrogen peroxide with a carboxylic acid is an equilibrium reaction, and the production of peracid is favored by the use of an excess concentration of peroxide and/or carboxylic acid, or by the removal of waster. There are several disadvantages to the chemical reaction for peracid production: a) the high concentration of carboxylic acid used to favor production of peracid can result in an undesirable odor when using the peracid-containing solution, 2) the peracid is oftentimes unstable in solution over time, and the concentration of peracid in the solution decreases during storage prior to use, and 3) the formulation is often strongly acidic due to the use of concentrated sulfuric acid as catalyst.

One way to overcome the disadvantages of the chemical production of peracids is to employ an enzyme catalyst in place of a strong acid catalyst. The use of an enzyme catalyst allows for the rapid production of peracid at the time of use and/or application, avoiding problems associated with storage of peracid solutions and variations in peracid concentrations over time. The high concentrations of carboxylic acids typically used to produce peracid via the direct chemical reaction with hydrogen peroxide are not required for enzymatic production of peracid, where the enzyme catalyzed reaction can use a carboxylic acid ester substrate at a much lower concentration than is typically used in the chemical reaction. The enzyme reaction can be performed across a broad range of pH, dependent on enzyme activity and stability at a given pH, and on the substrate specificity for perhydrolysis at a given pH.

Esterases, lipases, and some proteases have the ability to catalyze the hydrolysis of alkyl esters to produce the corresponding carboxylic acids (Formula 1).

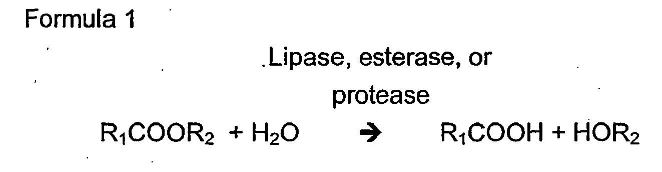

Some esterases, lipases and proteases exhibit perhydrolysis activity, catalyzing the synthesis of peracids from alkyl esters (Formula 2).

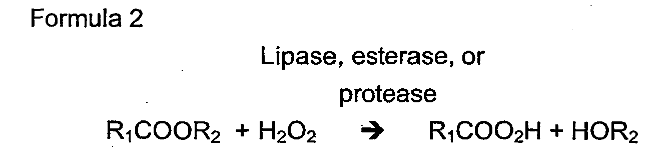

O. Kirk et al. (Biocatalysis, 11:65-77 (1994)) investigated the ability of hydrolases (lipases, esterases, and proteases) to catalyze perhydrolysis of acyl substrates with hydrogen peroxide to form peroxycarboxylic acids, and reported that perhydrolysis proceeds with a very low efficiency in aqueous systems. Furthermore, they found that lipases and esterases degraded percarboxylic acid to the corresponding carboxylic acid and hydrogen peroxide. The authors concluded that esterases, lipases and proteases are, in general, not suitable for catalyzing perhydrolysis of simple esters, such as methyl octanoate and trioctanoin, in an aqueous environment.

US 3,974,082 describes the production of bleaching compositions for laundry detergent applications by contacting the material to be bleached with an aqueous solution containing an oxygen-releasing inorganic peroxygen compound, an acyl alkyl ester, and an esterase or lipase capable of hydrolyzing the ester.

US 5,364,554 describes an activated oxidant system for *in situ* generation of peracid in aqueous solution using a protease enzyme, a source of hydrogen peroxide, and an ester substrate that is preferably chemically non-perhydrolyzable. A method of bleaching and a method of forming peracid are also disclosed.

US 5,296,161 describes production of peracid in an aqueous solution comprising one or more specific esterases and lipases, a source of hydrogen peroxide, and a functionalized ester substrate suitable for use in a bleaching composition. However, the concentration of peracid produced was generally insufficient for use in many commercial disinfectant applications.

Most known methods for preparing peracids from the corresponding carboxylic acid esters using enzyme catalysts do not produce and accumulate a peracid at a sufficiently-high concentration to be efficacious for disinfection in a variety of applications. Several protease and lipase combinations have recently been reported to generate peracids (e.g. peracetic acid) *in situ* at concentrations suitable for use as a disinfectant and/or commercial bleaching agent (US 11/413,246). However, there remains a need to identify additional enzyme catalysts capable of producing peracids *in situ.*

Haloperoxidases are widespread in nature, commonly found in animals, plants, bacteria, fungi, and algae. The first discovered haloperoxidases were reported to contain heme as a prosthetic group or cofactor. Subsequently, many non-heme haloperoxidases have been identified, primarily from bacteria.

Haloperoxidases catalyze the oxidation of halide ion (X= Cl, Br, or I) in the presence of hydrogen peroxide to the corresponding hypohalous acid (HOX), a reactive oxygen species that has been reported to have better antimicrobial activity than hydrogen peroxide (Formula 3).

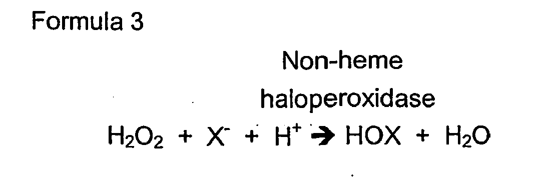

Plants recombinantly expressing a non-heme haloperoxidase from *Pseudomonas pyrrocinia* have been report to have improved pathogen resistance (Jacks et al., US 6,703,540). Jacks *et al.* report that the recombinantly expressed non-heme haloperoxidase also exhibited the ability to catalyze a reaction between acetic acid and hydrogen peroxide, forming the antimicrobial agent peracetic acid. Increased production of hypohalites and peracetic acid in the transgenic plant were correlated with the increase in pathogen resistance. However, it was later reported that the increased resistance to the fungal pathogen *Aspergillus flavus* in transgenic plants expressing the *P*. *pyrrocinia* non-heme haloperoxidase was probably not due to peracetic acid formation as the concentration of the substrates (hydrogen peroxide or acetate) needed for enzyme saturation was lethal (to the fungal pathogen) prior to its oxidation to peracetate (Jacks et al., J. Agric. Food Chem. 48:4561-4564 (2000); Jacks et al., J. Agric. Food Chem., 50:706-709 (2002)).

The problem to be solved is to provide a process to enzymatically produce peracids *in situ* at concentrations suitable for use in a variety of disinfectant applications. Preferably, the substrates used to produce the peracid compositions should be relatively non-toxic and inexpensive, such as carboxylic acid esters.

### SUMMARY OF THE INVENTION

The stated problem has been solved by the discovery that non-heme haloperoxidases, in the presence of an inorganic source of peroxygen (e.g. hydrogen peroxide) and a carboxylic acid ester substrate, produce concentrations of peracids *in situ* sufficient for use as a disinfectant and/or bleaching agent.

In one aspect of the invention, an aqueous enzymatic process for *in situ* generation of peracids using a non-heme haloperoxidase in combination with selected substrates is provided comprising:
a) providing a set of peracid reaction components, said components comprising:
   1. a substrate selected from the group consisting:
      i) esters having the structure 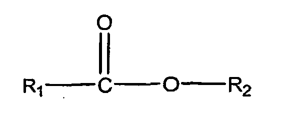 wherein R₁= C1 to C10 straight chain or branched chain alkyl optionally substituted with an hydroxyl or C1 to C4 alkoxy group and R₂= C1 to C10 straight chain or branched chain alkyl group, (CH₂CH₂-O)ₙH or (CH₂CH(CH₃)-O)ₙH and n=1 to 10;
      ii) glycerides having the structure 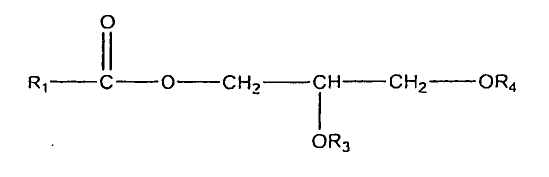 wherein R₁= C1 to C10 straight chain or branched chain alkyl optionally substituted with an hydroxyl or a C1 to C4 alkoxy group and R₃ and R₄ are individually H or R₁C(O);
   2) a source of peroxygen; and
   3) a non-heme haloperoxidase catalyst having perhydrolysis activity wherein the non-heme haloperoxidase having perhydrolysis activity is encoded by an isolated nucleic acid molecule selected from the group consisting of:
      a) an isolated nucleic acid molecule encoding a polypeptide having an amino acid sequence selected from the group consisting of SEQ ID NO: 10, SEQ ID NO: 14, SEQ ID NO: 18, SEQ ID NO: 22, and SEQ ID NO: 28; and
      b) an isolated nucleic acid molecule encoding a polypeptide having at least 95% identity to an amino acid sequence selected from the group consisting of SEQ ID NO: 10, SEQ ID NO: 14, SEQ ID NO: 18, SEQ ID NO: 22, and SEQ ID NO: 28;
      and
b) combining said reaction components under aqueous reaction conditions, wherein said conditions comprising a pH range of about 2 to about 7.5, whereby a peracid is produced at a concentration of at least 500 ppb within about 5 minutes to about 2 hours of combining the reaction components.

In another aspect of the invention, a process is provided to reduce a viable microbial population on a hard surface or inanimate object by contacting the peracid composition produced by the above process with a hard surface or inanimate object within 48-hours of mixing the peracid reaction components whereby the viable microbial population is reduced at least 3-log, preferably 4-log, more preferably at least 5-log, and most preferably at least 6-log. In a further aspect, the peracid product produced by the above methods may be optionally diluted to a desired efficacious concentration prior to contacting the surface or inanimate object to be treated.
In a further aspect of the invention an isolated nucleic acid molecule encoding a polypeptide having perhydrolytic activity wherein said polypeptide comprises an amino acid sequence having at least 98% identity to SEQ ID NO: 10 is also provided.

### BRIEF DESCRIPTION OF THE BIOLOGICAL SEQUENCES

The following sequences comply with 37 C.F.R. 1.821-1.825 ("Requirements for Patent Applications Containing Nucleotide Sequences and/or Amino Acid Sequence Disclosures - the Sequence Rules") and are consistent with World Intellectual Property Organization (WIPO) Standard ST.25 (1998) and the sequence listing requirements of the EPC and PCT (Rules 5.2 and 49.5(a-bis), and Section 208 and Annex C of the Administrative Instructions). The symbols and format used for nucleotide and amino acid sequence data comply with the rules set forth in 37 C.F.R. §1.822.
SEQ ID NO: 1 is the nucleotide sequence of a primer#1.
SEQ ID NO: 2 is the nucleotide sequence of a primer#2.
SEQ ID NO: 3 is the nucleotide sequence of a primer#3.
SEQ ID NO: 4 is the nucleotide sequence amplified from *Psuedomonas putida* 5B (NRRL-18668) genomic DNA using primer#1 and primer#2.
SEQ ID NO: 5 is the nucleotide sequence amplified from *Psuedomonas putida* 5B (NRRL-18668) genomic DNA using primer#1 and primer#3.
SEQ ID NO: 6 is the nucleotide sequence of a primer#4.
SEQ ID NO: 7 is the nucleotide sequence of a primer#5.
SEQ ID NO: 8 is the nucleotide sequence of a primer#6.
SEQ ID NO: 9 is the nucleotide sequence of the coding region of a non-heme haloperoxidase from *Pseudomonas putida* 5B (NRRL-18668).
SEQ ID NO: 10 is the deduced amino acid sequence of the non-heme haloperoxidase from *P. putida* 5B (NRRL-18668).
SEQ ID NO: 11 is the nucleotide sequence of a primer#7.
SEQ ID NO: 12 is the nucleotide sequence of a primer#8.
SEQ ID NO: 13 is the nucleotide sequence of a non-heme haloperoxidase ("AtuA1 ") from *Agrobacterium tumefaciens* C58 (GenBank® 16119616).
SEQ ID NO: 14 is the amino acid sequence of a non-heme haloperoxidase ("AtuA1 ") from *Agrobacterium tumefaciens* C58 (GenBank® 16119616).
SEQ ID NO: 15 is the nucleotide sequence of a primer#9.
SEQ ID NO: 16 is the nucleotide sequence of a primer#10.
SEQ ID NO: 17 is the nucleotide sequence of a non-heme haloperoxidase ("AtuA2") from *Agrobacterium tumefaciens* C58 (GenBank® 16119293).
SEQ ID NO: 18 is the amino acid sequence of a non-heme haloperoxidase ("AtuA2") from *Agrobacterium tumefaciens* C58 (GenBank® 16119293).
SEQ ID NO: 19 is the nucleotide sequence of a primer#11.
SEQ ID NO: 20 is the nucleotide sequence of a primer#12.
SEQ ID NO: 21 is the nucleotide sequence of a non-heme haloperoxidase ("AtuA3") from *Agrobacterium tumefaciens* C58 (GenBank® 15891475).
SEQ ID NO: 22 is the amino acid sequence of a non-heme haloperoxidase ("AtuA3") from *Agrobacterium tumefaciens* C58 (GenBank® 15891475).
SEQ ID NO: 23 is the nucleotide sequence of a primers#13.
SEQ ID NO: 24 is the nucleotide sequence of a primer#14.
SEQ ID NO: 25 is the nucleotide sequence of a primer#15.
SEQ ID NO: 26 is the nucleotide sequence of a primer#16.
SEQ ID NO: 27 is the nucleotide sequence of a non-heme haloperoxidase ("AtuA4") from *Agrobacterium tumefaciens* C58 (GenBank® 15891444).
SEQ ID NO: 28 is the amino acid sequence of a non-heme haloperoxidase ("AtuA4") from *Agrobacterium tumefaciens* C58 (GenBank® 15891444).
SEQ ID NO: 29 is the nucleotide sequence of a primer#17.
SEQ ID NO: 30 is the nucleotide sequence of a primer#18.
SEQ ID NO: 31 is the nucleotide sequence of a primer#19.
SEQ ID NO: 32 is the nucleotide sequence of a primer#20.

### DETAILED DESCRIPTION OF THE INVENTION

An aqueous enzymatic process for *in situ* generation of peracids from a carboxylic acid ester substrate using a non-heme haloperoxidase having perhydrolase activity is provided herein.

In one aspect of the invention, a process is provided as claimed in claim 8 whereby the viable microbial population is reduced at least 3-log, preferably 4-log, more preferably at least 5-log, and most -preferably at least 6-log.

In this disclosure, a number of terms and abbreviations are used. The following definitions apply unless specifically stated otherwise.

As used herein, the term "comprising" means the presence of the stated features, integers, steps, or components as referred to in the claims, but that it does not preclude the presence or addition of one or more other features, integers, steps, components or groups thereof.

As used herein, the term "peracid" is synonymous with peroxyacid, peroxy acid, percarboxylic acid and peroxoic acid.

As used herein, the term "peracetic acid" is abbreviated as "PAA" and is synonymous with peroxyacetic acid, ethaneperoxoic acid and all other synonyms of CAS Registry Number 79-21-0.

As used herein, the term "a source of peroxygen" means a peroxygen compound (i.e., a "peroxygen source") capable of providing hydrogen peroxide to the aqueous reaction mixture selected from the group consisting of perborate salts, percarbonate salts, perphosphate salts, hydrogen peroxide, hydrogen peroxide-urea adduct (CAS# 124-43-6), and mixtures thereof.

As used herein, the term "monoacetin" is synonymous with glycerol monoacetate, glycerin monoacetate, and glyceryl monoacetate.

As used herein, the term "diacetin" is synonymous with glycerol diacetate; glycerin diacetate, glyceryl diacetate, and all other synonyms of CAS Registry Number 25395-31-7.

As used herein, the term "triacetin" is synonymous with glycerin triacetate; glycerol triacetate; glyceryl triacetate, 1,2,3-triacetoxypropane, 1,2,3-propanetriol triacetate and all other synonyms of CAS Registry Number 102-76-1.

As used herein, the term "monobutyrin" is synonymous with glycerol monobutyrate, glycerin monobutyrate, and glyceryl monobutyrate.

As used herein, the term "dibutyrin" is synonymous with glycerol dibutyrate and glyceryl dibutyrate.

As used herein, the term "tributyrin" is synonymous with glycerol tributyrate, 1,2,3-tributyrylglycerol, and all other synonyms of CAS Registry Number 60-01-5.

As used herein, the term "monopropionin" is synonymous with glycerol monopropionate, glycerin monopropionate, and glyceryl monopropionate.

As used herein, the term "dipropionin" is synonymous with glycerol dipropionate and glyceryl dipropionate.

As used herein, the term "tripropionin" is synonymous with glyceryl tripropionate, glycerol tripropionate, 1,2,3-tripropionylglycerol, and all other synonyms of CAS Registry Number 139-45-7.

As used herein, the term "ethyl acetate" is synonymous with acetic ether, acetoxyethane, ethyl ethanoate, acetic acid ethyl ester, ethanoic acid ethyl ester, ethyl acetic ester and all other synonyms of CAS Registry Number 141-78-6.

As used herein, the term "ethyl lactate" is synonymous with lactic acid ethyl ester and all other synonyms of CAS Registry Number 97-64-3.

As used herein, the term "suitable aqueous reaction conditions " refers to the conditions in which the reactants and enzyme catalyst come into contact. The components and conditions suitable for the reaction are provided herein and those skilled in the art appreciate the range of component and condition variations suitable for these processes.

As used herein, the term "perhydrolysis" is defined as the reaction of a selected substrate with peroxide to form a peracid. Typically, an inorganic peroxide is reacted with the selected substrate in the presence of a catalyst to produce the peracid. As used herein, the term "chemical perhydrolysis" includes perhydrolysis reactions in which a substrate (a peracid precursor) is combined with a source of hydrogen peroxide wherein peracid is formed in the absence of an enzyme catalyst. As used herein, the term "enzymatic perhydrolysis" refers to a perhydrolysis reaction that is assisted or catalyzed by an enzyme.

As used herein, "non-heme haloperoxidase", "haloperoxidase", "HPO", and "bacterial haloperoxidase" refer to a non-heme haloperoxidase that may be further classified as a chloroperoxidase (E.C. 1.11.1 .10), bromoperoxidase, or iodoperoxidase (E.C. 1.11.1.8) that catalyzes the halogenation of a variety of substrates in the presence of halide ions and hydrogen peroxide. Non-heme haloperoxidases have been shown to catalyze the peroxidation of acyl carboxylic acids to peracids (US 6,703,540; Jacks *et al., supra,* 2000; Jacks *et al. supra,* 2002). As described herein, several non-heme haloperoxidases are provided that exhibit perhydrolysis activity under suitable aqueous reaction conditions towards a variety of carboxylic acid ester substrates.

As used herein, *"Pseudomonas putida* 5B" refers to the *Pseudomonas putida* strain deposited with the Northern Regional Research Lab (NRRL) under depository designation NRRL-1866.8 (US 5,811,286). The Northern Regional Research Lab has been renamed as the National Center for Agriculture, USDA Research (Peoria, IL).

As used herein, *"Agrobacterium tumefaciens"* refers to *Agrobacterium tumefaciens* strain C58 (Goodner et al., Science, 294(5550):2323-2328 (2001)). This bacterial strain is also referred to as *Rhizobium radiobacter* and is available from the American Type Culture Collection (ATCC®) under depository designation number ATCC 33970.

As used herein, the term "perhydrolase catalyst" refers herein to an enzyme catalyst (non-heme haloperoxidase) that is characterized by perhydrolysis activity. The enzyme catalyst may be in the form of a whole microbial cell, permeabilized microbial cell(s), one or more cell components of a microbial cell extract, partially purified enzyme, or purified enzyme. As described herein, non-heme haloperoxidases are shown to have perhydrolysis activity towards carboxylic acid esters.

As used herein, the term "perhydrolase activity" refers to the enzyme activity per unit mass (for example, milligram) of protein, dry cell weight, or immobilized catalyst weight. Comparisons of perhydrolase activity of catalysts were determined proportional to the dry cell weight or protein catalyst weight.

As used herein, "one unit of enzyme activity" or "one unit of activity" or "U" is defined as the amount of enzyme activity required for the production of 1 µmol of peracid product per minute at a specified temperature.

As used herein, the term "disinfect" refers to the process of cleansing so as to destroy and prevent the growth of pathogenic microorganisms. As used herein, the term "disinfectant" refers to an agent that disinfects by destroying, neutralizing, or inhibiting the growth of disease-carrying microorganisms and viruses. Typically disinfectants are used to treat inanimate objects or surfaces. As used herein, the term "antiseptic" refers to a chemical agent that inhibits the growth of disease-carrying microorganisms.

As used herein, the terms "virucide" and "viricide" refer to an agent that inhibits or destroys viruses. An agent that exhibits the ability to inhibit or destroy viruses is described as having "virucidal" or "viricidal" activity. Peracids can have virucidal activity. Typical alternative virucides known in the art which may be suitable for use with the present invention include, for example, alcohols, ethers, chloroform, formaldehyde, phenols, beta propiolactone, iodine, chlorine, mercury salts, hydroxylamine, ethylene oxide, ethylene glycol, quaternary ammonium compounds, enzymes, and detergents.

As used herein, the term "biocide" refers to a chemical agent, typically broad spectrum, that inactivates or destroys microorganisms. A chemical agent that exhibits the ability to inactivate or destroy microorganisms is described as having "biocidal" activity. Peracids can have biocidal activity. Typical alternative biocides known in the art, which may be suitable for use in the present invention include, for example, chlorine, chlorine dioxide, chloroisocyanurates, hypochlorites, ozone, acrolein, amines, chlorinated phenolics, copper salts, organo-sulphur compounds, and quaternary ammonium salts.

As used herein, the phrase "minimum biocidal concentration" refers to the minimum concentration of a biocidal agent that, for a specific contact time, will produce a desired lethal, irreversible reduction in the viable population of the targeted microorganisms. The effectiveness can be measured by the log₁₀ reduction in viable microorganisms after treatment. In one aspect, the targeted reduction in viable cells after treatment is a 3-log reduction, more preferably a 4-log reduction, and most preferably at least a 5-log reduction. In another aspect, the minimum biocidal concentration is a 6-log reduction in viable microbial cells.

As used herein, the term "about" modifying the quantity of an ingredient or reactant of the invention employed refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the compositions or carry out the methods; and the like. The term "about" also encompasses amounts that differ due to different equilibrium conditions for a composition resulting from a particular initial mixture.

### Suitable Reaction Conditions for the Enzyme-catayzed Preparation of Peracids from Carboxylic Acid Esters and Hydrogen Peroxide

In one aspect of the invention, a method is provided to produce an aqueous mixture comprising a peracid by reacting carboxylic acid esters and a source of peroxygen, e.g. an inorganic peroxide, not limited to hydrogen peroxide, sodium perborate or sodium percarbonate, in the presence of a non-heme haloperoxidase catalyst having perhydrolysis activity, as claimed in claim 1.

The carboxylic acid ester substrates have a formula selected from the group consisting of:
a) esters of the formula 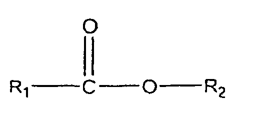 wherein R₁= C1 to C10 straight chain or branched chain alkyl optionally substituted with an hydroxyl or a C1 to C4 alkoxy group and R₂= C1 to C10 strain chain or branched chain alkyl group, (CH₂CH₁-O)ₙH or (CH₂CH(CH₃)-O)ₙH and n=1 to 10; and
b) glycerides of the formula 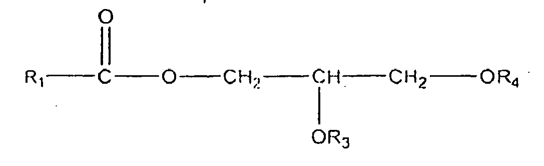 wherein R₁= C1 to C10 straight chain or branched chain alkyl optionally substituted with an hydroxyl or a C1 to C4 alkoxy group and R₃ and R₄ are individually H or R₁C(O).

In one aspect, the carboxylic acid ester substrates are selected from the group consisting of methyl lactate, ethyl lactate, methyl glycolate, ethyl glycolate, methyl methoxyacetate, ethyl methoxyacetate, methyl 3-hydroxybutyrate, ethyl 3-hydroxybutyrate, triethyl 2-acetyl citrate, glucose pentaacetate, gluconolactone, glycerides (mono-, di-, and triglycerides) such as monoacetin, diacetin (1,2,3-propanetriol diacetate), triacetin (glyceryl triacetate), monopropionin, glyceryl dipropionate, tripropionin (glycercyl tripropionate), monobutyrin, dibutyrin (glyceryl dibutyrate), tributyrin (glyceryl tributyrate), and mixtures thereof.

In another aspect, the carboxylic acid ester substrates are selected from the group consisting of monoacetin, diacetin, triacetin, glyceryl dipropionate, tripropionin, monobutyrin, dibutyrin, tributyrin, ethyl acetate, and ethyl lactate. In yet another aspect, the carboxylic acid ester substrates are selected from the group consisting of diacetin, triacetin, ethyl acetate, and ethyl lactate.

The enzyme substrate (carboxylic acid ester) is present in the reaction mixture at a concentration sufficient to produce the desired concentration of peracid upon enzyme-catalyzed perhydrolysis. It has been demonstrated in the accompanying examples that there are preferred combinations of enzyme substrate and enzyme catalyst that produce a desirable concentration of peracid. The carboxylic acid ester need not be completely soluble in the reaction mixture, but have sufficient solubility to permit conversion of the ester by the enzyme catalyst to the corresponding peracid. The carboxylic acid ester is preferably present in the reaction mixture at a concentration of 0.05 wt% to 40 wt % of the reaction mixture, more preferably at a concentration of 0.1 wt % to 20 wt % of the reaction mixture, and most preferably at a concentration of 0.5 wt % to 10 wt % of the reaction mixture.

The peroxygen source may include, but is not limited to, hydrogen peroxide, perborate salts, perphosphate salts, percarbonate salts, and mixtures thereof. The concentration of peroxygen compound in the reaction mixture may range from 0.1 wt % to about 50 wt %, preferably from 1 wt % to about 40 wt %, more preferably from 2 wt % to about 30 wt %.

The enzyme catalysts described herein are members of the non-heme haloperoxidase family (for example, chloroperoxidases [E.C. 1.11.1.10], bromoperoxidases, and iodoperoxidases [E.C. 1.11.1.8]). A catalytic triad (serine 97:aspartic acid 229:histidine 258) has been reported to be responsible for the halogenation activity in this family of proteins (Pelletier et al., Biochimica et Biophysica Acta 1250:149-157 (1995)). The present non-heme haloperoxidases are comprised of this triad, although one of the present non-heme haloperoxidases from *Agrobacterium tumefaciens* (AtuA2; SEQ ID NO: 18) appears to have a 47 amino acid N-terminal extension. Absent the 47 amino acid N-terminal extension, AtuA2 is comprised of the characteristic triad responsible for halogenation activity. In particular, the enzymes that are useful in the present invention are non-heme haloperoxidases having perhydrolysis activity towards carboxylic acid ester substrates.

In one aspect, the enzyme catalyst is a non-heme haloperoxidase derived or isolated from a prokaryotic organism. In another aspect, the non-heme haloperoxidase is derived or isolated from a bacterium. In another aspect, the non-heme haloperoxidase is derived from an organism selected from the genera consisting of Pseudomonas sp. and Agrobacterium sp. In a further aspect, the source of the non-heme haloperoxidase is selected from the group consisting of Pseudomonas putida and Agrobacterium tumefaciens. In a preferred aspect, the source of the non-heme haloperoxidase is selected from the group consisting of Pseudomonas putida 5B (NRRL-18668) and Agrobacterium tumefaciens C58 (ATCC 33970).

Nucleic acid molecules which may be used to produce the non-heme haloperoxidase catalysts include those encoding non-heme haloperoxidases having at least 95% identity to an amino acid sequence selected from the group consisting of SEQ ID NO: 10, 14, 18, 22, and 28. In another aspect, nucleic acid molecules encoding non-heme haloperoxidases useful in the present process have preferably at least 98%, and most preferably at least 99% identity to the above amino acid sequences. The nucleic acid sequences encoding non-heme haloperoxidases useful in the present process preferably encode an amino acid sequence selected from the group consisting of SEQ ID NOs: 10, 14, 18, 22, and 28.

Also disclosed are nucleic acid sequences encoding non- heme haloperoxidases suitable in the present process have at least 80%, more preferably at least 85%, even more preferably at least 90%, yet even more preferably 95%, even yet more preferably 98%, and more preferably at least 99% nucleic acid sequence identity to the sequences provided herein. Preferably nucleic acid sequences encoding non-heme haloperoxidases suitable in the present process have a nucleic acid sequence selected from the group consisting of SEQ ID NOs: 9, 13, 17, 21, and 27.

Many enzyme catalysts (whole cell, partially purified, or purified) have been reported to have catalase activity (EC 1.11.1.6). Catalases catalyze the conversion of hydrogen peroxide into oxygen and water. In one aspect, the perhydrolysis catalyst lacks catalase activity. In another aspect, a catalase inhibitor is added to the reaction mixture. Examples of catalase inhibitors include, but are not limited to, sodium azide and hydroxylamine sulfate. One of skill in the art can adjust the concentration of catalase inhibitor as needed. The concentration of the catalase inhibitor typically ranges from 0.1 mM to about 1 M; preferably about 1 mM to about 50 mM; more preferably from about 1 mM to about 20 mM. In one aspect, sodium azide concentration typically ranges from about 20 mM to about 60 mM while hydroxylamine sulfate concentration is typically about 0.5 mM to about 30 mM, preferably about 10 mM. In a preferred embodiment, the enzyme catalyst lacks significant catalase activity or is engineered to decrease or eliminate catalase activity. Host cells used for recombinant expression of the present perhydrolytic enzymes can be selected that lack catalase activity (for example, *E. coli* strain UM2 (CGSC 7156)). In a further embodiment, the catalase activity in a host cell can be down-regulated or eliminated by disrupting expression of the gene(s) responsible for the catalase activity using well known techniques including, but not limited to, transposon mutagenesis, RNA antisense expression, targeted mutagenesis, and random mutagenesis.

The concentration of enzyme catalyst in the aqueous reaction mixture is chosen to obtain the desired rate of reaction, and depends on the specific catalytic activity of the enzyme catalyst. The weight of a soluble enzyme used as catalyst in perhydrolysis reactions typically ranges from 0.05 mg to 10 mg of enzyme per mL of total reaction volume, preferably from 0.10 mg to 2.0 mg of enzyme per mL. The enzyme may also be immobilized on a soluble or insoluble support using methods well-known to those skilled in the art; see for example, Immobilization of Enzymes and Cells; Gordon F. Bickerstaff, Editor; Humana Press, Totowa, NJ, USA; 1997. The use of immobilized enzymes permits the recovery and reuse of the catalyst in subsequent reactions. Additional forms of the enzyme catalyst that are useful in the present application include whole microbial cells, cell extracts, and partially-purified enzymes. These additional forms of the catalysts may also be immobilized using the methods referenced above.

In one aspect, the concentration of peracid generated by the combination of chemical perhydrolysis and enzymatic perhydrolysis of the carboxylic acid ester is sufficient to provide an effective concentration of peracid for bleaching or disinfection at a desired pH. In another aspect, the present methods provide combinations of enzymes and enzyme substrates to produce the desired effective concentration of peracid, where, in the absence of added enzyme, there is a significantly-lower concentration of peracid produced. Although there may in some cases be significant chemical perhydrolysis of the enzyme substrate by direct chemical reaction of inorganic peroxide with the enzyme substrate, there may not be a sufficient concentration of peracid generated by chemical perhydrolysis to provide an effective concentration of peracid in the desired applications, and a significant increase in total peracid concentration is achieved by the addition of an appropriate enzyme catalyst to the reaction mixture.

The concentration of peracid generated by enzyme-catalyzed perhydrolysis of carboxylic acid ester is at least 500 ppb peracid, preferably at least 1 ppm peracid, more preferably at least 2 ppm peracid, even more preferably at least 4 ppm peracid, even yet more preferably at least 5 ppm, and most preferably at least 8 ppm. The product mixture comprising the peracid may be optionally diluted with water, or a solution predominantly comprised of water, to produce a mixture with the desired lower concentration of peracid. The reaction time required to produce the desired concentration of peracid is not greater than about two hours, preferably not greater than about 30 minutes, more preferably not greater than about 10 minutes, and most preferably less than about 5 minutes. The peracid concentration may continue to increase above the initial desired concentration of peracid generated in not greater than about 2 hours, such that the reaction mixture produces a maximum concentration of peracid in at least 48 hours.

The temperature of the reaction is chosen to control both the reaction rate and the stability of the enzyme catalyst activity. The temperature of the reaction may range from just above the freezing point of the reaction mixture (approximately 0 °C) to about 65 °C, with a preferred range of reaction temperature of from about 5 °C to about 35 °C.

The pH of the final reaction mixture containing peracid is from about 2 to about 7.5, preferably from about 3 to about 7, and most preferably about 3.5 to about 6.5. The pH of the reaction, and of the final reaction mixture, may be controlled by the addition of a suitable buffer, including, but not limited to phosphate, pyrophosphate, bicarbonate, acetate, or citrate. The concentration of buffer is from 0.1 mM to 1.0 M, preferably from 1 mM to 100 mM, most preferably from 10 mM to 50 mM.

In another aspect, the enzymatic perhydrolysis product may contain additional components that provide desirable functionality. In one aspect, the desirable functionality may include use of the present materials in bleaching applications. These additional components include, but are not limited to emulsifiers and surfactants. Examples of emulsifiers include polyvinyl alcohol or polyvinylpyrrolidine. Examples of surfactants, including a) non-ionic surfactants such as block copolymers of ethylene oxide or propylene oxide, ethoxylated or propoxylated linear and branched primary and secondary alcohols, and aliphatic phosphine oxides b) cationic surfactants such as such as quaternary ammonium compounds, particularly quaternary ammonium compounds having a C8-C20 alkyl group bound to a nitrogen atom additionally bound to three C1-C2 alkyl groups, c) anionic surfactants such as alkane carboxylic acids (e.g., C8-C20 fatty acids), alkyl phosphonates, alkane sulfonates (e.g., sodium dodecylsulphate) or linear or branched alkyl benzene sulfonates, alkene sulfonates and d) amphoteric and zwitterionic surfactants such as aminocarboxylic acids, aminodicarboxylic acids, and alkybetaines. Additional components may include fragrances, dyes, stabilizers of hydrogen peroxide (e.g., 1-hydroxyethylidene -1,1,-diphosphonic acid (Dequest 2010, Solutia Inc., St. Louis, MO)), stabilizers of enzyme activity (e.g., polyethyleneglycol (PEG)), detergent builders and metal chelators (e.g., ethylenediaminetetraacetic acid (EDTA)).

### Enzymatic In Situ Production of Peracids

The present aqueous enzymatic method produces useful concentrations of peracids for biocidal and/or virucidal applications *in situ.* The peracids produced are quite reactive and unstable, generally decreasing in concentration over time. As such, it may be desirable to keep the various reaction components separated, especially for formulations where at least one component of the formulation is a liquid. In one aspect, the hydrogen peroxide source is separate from either the substrate or the enzyme, preferably from both. This can be accomplished using a variety of techniques including, but not limited to the use of multicompartment chambered dispensers (US 4,585,150) and subsequently physically combining the enzyme catalyst with the present substrates to initiate the aqueous enzymatic perhydrolysis reaction. The enzyme catalyst may be immobilized within the body of reaction chamber or separated (e.g. filtered, etc.) from the reaction product comprising the peracid prior to contacting the surface and/or object targeted for treatment. The enzyme catalyst may be in a liquid matrix or in a solid form (i.e. powdered, tablet) or embedded within a solid matrix that is subsequently mixed with the substrates to initiate the enzymatic perhydrolysis reaction. In a further aspect, the enzyme catalyst may be contained within a dissolvable or porous pouch that may be added to the aqueous substrate matrix to initiate enzymatic perhydrolysis.

### HPLC Assay Method for Determining the Concentration of Peracid and Hydrogen Peroxide.

A variety of analytical methods can be used in the present method to analyze the reactants and products including, but not limited to titration, high performance liquid chromatography (HPLC), gas chromatography (GC), mass spectroscopy (MS), capillary electrophoresis (CE), the analytical procedure described by U. Karst et al., (Anal. Chem., 69(17):3623-3627 (1997)), and the 2,2'-azino-bis (3-ethylbenzothazoline)-6-sulfonate (ABTS) assay (S. Minning, et al., Analytica Chimica Acta 378:293-298 (1999) and WO 2004/058961 A1) as described in the present examples.

### Determination of Minimum Biocidal Concentration of Peracids

The method described by J. Gabrielson, et al. (J. Microbiol. Methods 50: 63-73 (2002)) can be employed for determination of the Minimum Biocidal Concentration (MBC) of peracids, or of hydrogen peroxide and enzyme substrates. The assay method is based on XTT reduction inhibition, where XTT ((2,3-bis[2-methoxy-4-nitro-5-sulfophenyl]-5-[(phenylamino)carbonyl]-2H-tetrazolium, inner salt, monosodium salt) is a redox dye that indicates microbial respiratory activity by a change in optical density (OD) measured at 490 nm or 450 nm. However, there are a variety of other methods available for testing the activity of disinfectants and antiseptics including, but not limited to viable plate counts, direct microscopic counts, dry weight, turbidity measurements, absorbance, and bioluminescence (see, for example Brock, Semour S., Disinfection, Sterilization, and Preservation, 5th edition, Lippincott Williams & Wilkins, Philadelphia, PA, USA; 2001).

### Uses of Enzymatically-Prepared Peracid Compositions

The enzyme-generated peracid produced according to the present methods can be used in a variety of applications for reduction of microbial, fungal, and viral contamination, such as decontamination of medical instruments (e.g., endoscopes), textiles (e.g., garments, carpets), food preparation surfaces, food storage and food-packaging equipment, materials used for the packaging of food products, chicken hatcheries and grow-out facilities, animal enclosures, and spent process waters that have microbial and/or virucidal activity. The enzyme-generated peracids may be used in formulations designed to inactivate prions (e.g. formulations containing certain proteases) to additionally provide biocidal activity. In a preferred aspect, the present peracid compositions are particularly useful as a cleaning and disinfecting agent for non-autoclavable medical instruments and food packaging equipment. As the peracid-containing formulation may be prepared using GRAS or food-grade components (enzyme, enzyme substrate, hydrogen peroxide, and buffer), the enzyme-generated peracid may also be used for decontamination of animal carcasses, meat, fruits and vegetables, or for decontamination of prepared foods. The enzyme-generated peracid may be incorporated into a product whose final form is a powder, liquid, gel, solid or aerosol. The enzyme-generated peracid may be diluted to a concentration that still provides an efficacious decontamination.

The compositions comprising an efficacious concentration of peracid can be used to clean and disinfect surfaces and/or objects contaminated (or suspected of being contaminated) with pathogenic microorganisms and/or viruses by contacting the surface or object with the products produced by the present processes. As used herein, "contacting" refers to placing a disinfecting composition comprising an effective concentration of peracid in contact with the surface or inanimate object suspected of contamination with a disease-causing entity for a period of time sufficient to clean and disinfect. Contacting includes spraying, treating, immersing, flushing, pouring on or in, mixing, combining, painting, coating, applying, affixing to and otherwise communicating a peracid solution comprising an efficacious concentration of peracid with the surface or inanimate object suspected of being contaminated. The compositions comprising an efficacious concentration of peracid can also contain an additional antimicrobial agent, virucide or biocide. Combinations of these agents with the peracid produced by the claimed processes can provide for increased and/or synergistic effects when used to clean and disinfect surfaces and/or objects contaminated (or suspected of being contaminated) with pathogenic microorganisms, viruses, and/or prions. Suitable antimicrobial agents include carboxylic esters (e.g., p-hydroxy alkyl benzoates and alkyl cinnamates), sulfonic acids (e.g., dodecylbenzene sulfonic acid), iodo-compounds or active halogen compounds (e.g., elemental halogens, halogen oxides (e.g., NaOCl, HOCI, HOBr, ClO₂), iodine, interhalides (e.g., iodine monochloride, iodine dichloride, iodine trichloride, iodine tetrachloride, bromine chloride, iodine monobromide, or iodine dibromide), polyhalides, hypochlorite salts, hypochlorous acid, hypobromite salts, hypobromous acid, chloro- and bromo-hydantoins, chlorine dioxide, and sodium chlorite), organic peroxides including benzoyl peroxide, alkyl benzoyl peroxides, ozone, singlet oxygen generators, and mixtures thereof, phenolic derivatives (e.g., o-phenyl phenol, o-benzyl-p-chlorophenol, tert-amyl phenol and C₁-C₆ alkyl hydroxy benzoates), quaternary ammonium compounds (e.g., alkyldimethylbenzyl ammonium chloride, dialkyldimethyl ammonium chloride and mixtures thereof), and mixtures of such antimicrobial agents, in an amount sufficient to provide the desired degree of microbial protection. Effective amounts of antimicrobial agents include about 0.001 wt-% to about 60 wt-% antimicrobial agent, about 0.01 wt-% to about 15 wt-% antimicrobial agent, or about 0.08 wt-% to about 2.5-wt-% antimicrobial agent.

Further, when an amount, concentration, or other value or parameter is given either as a range, preferred range, or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

### GENERAL METHODS

The following examples are provided to demonstrate preferred aspects of the invention. It should be appreciated by those of skill in the art that the techniques disclosed in the examples which follow represent techniques discovered by the inventors to function well in the practice of the invention, and thus can be considered to constitute preferred modes for its practice.

### PCR Parameters:

Methods to synthesize nucleic acid molecules using PCR amplification are well known in the art. PCR reagents were obtained from Roche Diagnostics Corporation (Indianapolis, IN) and used as recommended by the manufacturer's instructions.

PCR cycle parameters were are follows:
Step 1: 5 minutes at 95 °C
Step 2: 0.5 minutes at 95 °C (denaturation)
Step 3: 0.5 minutes at 55 °C (annealing)
Step 4: 1 minutes at 74 °C (extension)
Steps 2-4 are repeated 25 cycles

All reagents and materials were obtained from DIFCO Laboratories (Detroit, MI), GIBCO/BRL (Gaithersburg, MD), TCI America (Portland, OR), Roche Diagnostics Corporation (Indianapolis, IN) or Sigma/Aldrich Chemical Company (St. Louis, MO), unless otherwise specified.

The following abbreviations in the specification correspond to units of measure, techniques, properties, or compounds as follows: "sec" or "s" means second(s), "min" means minute(s), "h" or "hr" means hour(s), "d" means density in g/mL, "µL" means microliters, "mL" means milliliters, "L" means liters, "mM" means millimolar, "M" means molar, "mmol" means millimole(s), "ppm" means parts per million, "ppb" means parts per billion, "wt" means weight, "wt%" means weight percent, "g" means grams, "µg" means micrograms, HPLC" means high performance liquid chromatography, "O.D." means optical density at the designated wavelength, "dcw" means dry cell weight, "CFU" means colony forming units, "ATCC" or "ATCC®" means the American Type Culture Collection, "U" means units of perhydrolase activity, "RPM" means revolutions per minute, "EDTA" means ethylenediaminetetraacetic acid, "IPTG" means isopropyl β-D-1-thiogalactopyranoside, "CFU" means colony forming units, , "XTT" means ((2,3-Bis-[2-methoxy-4-nitro-5-sulfophenyl]-2H-tetrazolium-5-carboxanilide inner salt; CAS# 111072-31-2), and "CGSC" means Coli Genetic Stock Center (Yale University *Escherichia Coli* Genetic Stock Center, New Haven, CT).

### EXAMPLE 1

### Clowning of P. putida 5B non-heme haloperoxidase

A series of PCR primers were designed based on published non-heme haloperoxidase sequences from *P. putida* strains, including KT2440, IF-3, and MR-2068 (GenBank® 26991929, 6451702, and 1360922; respectively). PCR using the genomic DNA from *P. putida* 5B (NRRL-18668) with primer#1 (5-GATCTGGTCATCGTCGCCATGCATCAC-3'; SEQ ID NO: 1) and primer#2 (5'- GCCGACGACTGGGACGCGCAGATG-3'; SEQ ID NO: 2) and with primer#1 and primer#3 (5'-ATGAGCTACGTCACCACCAAAGATGG-3'; SEQ ID NO: 3) produced products of approximately 600 bp and 700 bp, respectively. Nucleotide sequences of these products (SEQ ID NOs: 4 and 5) and the corresponding deduced amino acid sequences confirmed the identity of a partial non-heme haloperoxidase gene. Genomic sequencing with primer#4 (5'- GGCTACGTCGTCGGCATAGTGGTC-3'; SEQ ID NO: 6) revealed the presence of a Pstl restriction site several hundred bp upstream of the non-heme haloperoxidase gene. Genomic DNA was digested with *Pst*l followed by ligation, and inverse PCR was performed using primer#5 (5'- GGTGGAAGTGGATCACCGGTGCATCGC-3'; SEQ ID NO: 7) and primer#6 (5'- GCCCATTACGATGGCATCGTGGC-3'; SEQ ID NO: 8). Nucleotide sequence obtained from this PCR product was used to elucidate the entire native *Psuedomonas putida* 5B non-heme haloperoxidase coding sequence (SEQ ID NO: 9) encoding the amino acid represented by SEQ ID NO: 10.

### EXAMPLE 2

### Expression of P. putida 5B non-heme haloperoxidase in E. coli

The non-heme haloperoxidase gene from *P. putida* 5B was PCR amplified using primer#7 (5'-GAATTCATGAGCTATGTAACCACGAAGGACGGC-3'; SEQ ID NO: 11)
and primer#8 (5'-GCGGCCGCTTAACTACGGATAAACGCCAGCAAATCCGCAT-3'; SEQ ID NO: 12), and subcloned into pTrcHis2-TOPO® (Invitrogen) and into pCR®4-TOPO® (Invitrogen) to generate the expression plasmids pSW167 and pSW169, respectively. In addition, the *EcoR*I fragment from pSW169 was subcloned into pET-28a (Novagen; Madison, WI) to generate the expression plasmid pSW174. *E. coli* TOP10 (Invitrogen) was transformed with pSW167 or pSW169 to generate the strains TOP10/pSW167 and TOP10/pSW169, respectively. *E. coli* BL21 (DE3) (Novagen) was transformed with pSW174 to generate the strain BL21/pSW174. The 5B non-heme haloperoxidase was expressed in TOP10/pSW167, TOP10/pSW169, and BL21/pSW174 according to Invitrogen's expression protocol. Essentially, cells were induced at OD₆₀₀ = 0.5-0.6 with 1 mM IPTG for 3 hr at 37°C with shaking. SDS-PAGE analysis confirmed production of a protein with molecular weight of approximately 30 kDa, a band not present in the control strain (host without plasmid). Also transformed with pSW167 and with pSW169-were *E. coli* FM5 (ATCC 53911) and *E. coli* UM2 (CGSC 7156) to generate the strains FM5/pSW167, UM2/pSW167, FM5/pSW169, and UM2/pSW169.

### EXAMPLE 3

### Expression of A. tumefaciens non-heme haloperoxidase (GenBank® 16119616) in E. coli

The non-heme haloperoxidase gene (GenBank® 16119616; herein also referred to as "AtuA1"; SEQ ID NOs: 13-14) from *A. tumefaciens* C58 ("C58") was PCR-amplified using primer#9 (5'-ATGGGCTTCGTAACAACCAAGGACGGCAC-3'; SEQ ID NO: 15) and primer#10 (5'- TCAGCCCTTGATGAAGGCTAGCAGGTCCTG-3'; SEQ ID NO: 16), and subcloned into pCR®4-TOPO® (Invitrogen) to generate the plasmid pSW166.. In addition, the *EcoR*I fragment from pSW166 was subcloned into pET-28a (Novagen) to generate the expression plasmid pSW175. *E. coli* TOP10 (Invitrogen) was transformed with pSW166 to generate the strain TOP10/pSW166. *E. coli* BL21 (DE3) (Novagen) was transformed with pSW175 to generate the strain BL21/pSW175. The C58 non-heme haloperoxidase was expressed in TOP10/pSW166 and BL21/pSW175 by inducing at OD₆₀₀ = 0.5-0.6 with 1 mM IPTG for 3 hr at 37°C with shaking. SDS-PAGE analysis confirmed production of a protein with molecular weight of approximately 30 kDa, a protein not present in the control strains (cells without plasmid). Also transformed with pSW166 were *E. coli* FM5 (ATCC 53911) and *E. coli* UM2 (CGSC 7156) to generate the strains FM5/pSW166 and UM2/pSW166.

### EXAMPLE 4

### Expression of A. tumefaciens non-heme haloperoxidase (GenBank® 16119293) in E. coli

The non-heme haloperoxidase gene (GenBank® 16119293; herein also referred to "AtuA2"; SEQ ID NOs: 17-18) from *A. tumefaciens* C58 was PCR-amplified using primer#11 (5'-ATGTCGCGTTTCACCACCACTGACGGTACC-3'; SEQ ID NO: 19 and primer#12 (5'-TCAGCCGTGGATAAAGGCGAGCACGTCG-3'; SEQ ID NO: 20, and subcloned into pCR®4-TOPO® (invitrogen) and into pTrcHis2-TOPO® (Invitrogen) to generate the expression plasmids pSW171 and pSW173, respectively. In addition, the *EcoR*I fragment from pSW171 was subcloned into pET-28a (Novagen) to generate the expression plasmid pSW176. *E. coli* TOP10 (Invitrogen) was transformed with pSW171 or pSW173 to generate the strains TOP10/pSW171 and TOP10/pSW173, respectively. *E. coli* BL21 (DE3) (Novagen) was transformed with pSW 176 to generate the strain BL21/pSW176. The C58 non-heme haloperoxidase was expressed in TOP10/pSW171, TOP10/pSW173, and BL21/pSW176 by inducing at OD₆₀₀ = 0.5-0.6 with 1 mM IPTG for 3 hr at 37°C with shaking. SDS-PAGE analysis confirmed production of a protein with molecular weight of approximately 30 kDa, a protein not present in the control strains (host cells without plasmid). Also transformed with pSW171 and pSW173 were *E. coli* FM5 (ATCC 53911) and *E. coli* UM2 (CGSC 7156) to generate the strains FMS/pSW171, UM2/pSW171 FM5/pSW173, and UM2/pSW173.

### EXAMPLE 5

### Expression of A. tumefaciens non-heme haloperoxidase (GenBank® 15891475) in E. coli

The non-heme haloperoxidase gene (GenBank® 15891475; herein also referred to "AtuA3"; SEQ ID NOs: 21-22) from *A. tumefaciens* C58 was PCR-amplified using primer#13 (5'-ATGACAACGATCACCACCAGAGACGGAACG-3'; SEQ ID NO: 23) and primer#14 (5'- TCAGGCGTTGATGAAGGTGAGGAGGTCG-3'; SEQ ID NO: 24), and subcloned into pTrcHis2-TOPO® (Invitrogen) to generate the plasmid pSW170. In addition, the non-heme haloperoxidase gene (GenBank® 15891475) from *A. tumefaciens* C58 was PCR amplified using primer#15 (5'-CAATTGATGACAACGATCACCACCAGAGACG-3'; SEQ ID NO: 25) and primter#16 (5'- CAATTGCGGCCGCTTAGGCGTTGATGAAGGTGAGGAGGTCGGC-3'; SEQ ID NO: 26), and subcloned into pET-28a (Novagen) between *Mfe*I and *Not*I to generate the expression plasmid pSW177. *E. coli* TOP10 (Invitrogen) was transformed with pSW170 to generate the strain TOP10/pSW170. The C58 non-heme haloperoxidase was expressed in TOP10/pSW170 and BL21/pSW177 by inducing at OD₆₀₀ = 0.5-0.6 with 1 mM IPTG for 3 hr at 37°C with shaking. SDS-PAGE analysis confirmed production of a protein with molecular weight of approximately 30 kDa, a protein not present in the control strains (host cells without plasmid). Also transformed with pSW170 were *E. coli* FM5 (ATCC 53911) and *E. coli* UM2 (CGSC 7156) to generate the strains FM5/pSW170and UM2/pSW170.

### EXAMPLE 6

### Expression of A. tumefaciens non-heme haloperoxidase (GenBank® 15891444) in E. coli

The non-heme haloperoxidase gene (GenBank® 15891444; herein also referred to "AtuA4"; SEQ ID NOs: 27-28) from *A. tumefaciens* C58 was PCR-amplified using primer#17 (5-ATGGGCTTTGTTAAGACGACAGACGGAACC-3'; SEQ ID NO: 29 and primer#18 (5'-TCAGCCCTTGATGAAGGCCAGCAGATCC-3'; SEQ ID NO: 30, and subcloned into pTrcHis2-TOPO® (Invitrogen) to generate the plasmid pSW172. In addition, the non-heme haloperoxidase gene (GenBank® 15891444) from *A. tumefaciens* C58 (C58) was PCR amplified using primer#19 (5- ATGAATTCATGGGCTTTGTTAAGACGACAGACG-3'; SEQ ID NO: 31) and primer#20 (5'-ATGCGGCCGCTCAGCCCTTGATGAAGGCCAGCAGATC-3'; SEQ ID NO: 32), and subcloned into pET-28a (Novagen) to generate the expression plasmid pSW178. *E. coli* TOP10 (Invitrogen) was transformed with pSW172 to generate the strain TOP10/pSW172. *E. coli* BL21 (DE3) (Novagen) was transformed with pSW 178 to generate the strain BL21/pSW178. The C58 non-heme haloperoxidase was expressed in TOP10/pSW172 and BL21/pSW178 by inducing at OD₆₀₀ = 0.5-0.6 with 1 mM IPTG for 3 hr at 37°C with shaking. SDS-PAGE analysis confirmed production of a protein with molecular weight of approximately 30 kDa, a protein not present in the control strains (host cells without plasmid). Also transformed with pSW172 were *E. coli* FM5 (ATCC 53911) and *E. coli* UM2 (CGSC 7156) to generate the strains FM5/pSW172and UM2/pSW172.

### EXAMPLE 7

### Preparation of Cell Paste

A single colony from a LB plate containing either ampicillin (*E. coli* TOP10) or kanamycin (*E. coli* BL21) was transferred to 2 mL of Miller's LB broth containing 50 µg/mL ampicillin or kanamycin and grown for 16-18 hours at 37°C with shaking (250 rpm). 50 mL of Miller's LB containing the appropriate antibiotic was inoculated with 0.5 mL of 18 hr culture. Cells were grown at 37 °C with shaking (250 rpm) until an optical density (OD₆₀₀) of 0.5 was reached. A 1 M solution of IPTG was added to a final concentration of 1 mM and growth continued for 3 hours. Cells were harvested by centrifugation at 7,000 rpm for 20 minutes. Wet cell weight was determined and cell pellet was frozen and stored at -70 °C. This method may be scaled to 500 mL.

Non-recombinant cell lines (*E. coli* and *Pseudomonas putida* 5B . (NRRL-18668)) were transferred from a single colony on LB agar with no antibiotics to 50 mL of Miller's LB. Growth was at 37°C with shaking (250 rpm) for 8-18 hours. Harvest conditions were the same as for recombinant cells.

### EXAMPLE 8

### Peracid Production and Detection Assay at pH 6.5

This example describes a method that combines the production of peracid and the measurement of the amount of peracid produced. The assay was carried out in 96-well microtiter plates and analyzed using a SpectroMax® Plus (Molecular Devices Corp.; Sunnyvale, CA) plate reader at 405 nm.

Oxidation of 2,2'-azino-bis-(3-ethylbenzothiazoline)-6-sulfonate (ABTS) was used to measure peracid concentration. Briefly, the enzyme catalyst was mixed with the substrate in the presence of hydrogen peroxide to produce the peracid. The amount of peracid produced was determined by mixing the peracid with ABTS. The concentration of the oxidized ABTS was measured spectrophotometrically at 405 nm in microtiter plates.

Whole cell paste was suspended in 0.1 M KH₂PO₄ buffer (pH 6.5) at a final concentration of 50 mg/mL wet cell weight. A mixture of 100 µL of cell suspension, 30 µL of 0.1 M phosphate buffer (pH 6.5), 20 µL of 313 mM hydrogen peroxide and 50 µL of 200 mM substrate were incubated at 37°C for 15 minutes to 24 hr. Control samples were prepared by omitting hydrogen peroxide from the reaction mixture, and substituting an equivalent volume of deionized water. The reaction mixture was assayed by mixing 100 µL of the supernatant from a centrifuged reaction mixture with 50 µL of 1.5 M acetic acid containing 0.03 mg/mL potassium iodide and 50 µL of 1 mg/mL 2,2'-azino-bis-(3-ethylbenzothiazoline)-6-sulfonate (ABTS) in a microtiter plate well, incubating at room temperature for 10 minutes, and measuring the absorbance of the assay mixture at 405 nm.

### EXAMPLE 9

### Peracid Production and Detection Assay at pH 4.0

This example describes a method that combines the production of peracid and the measurement of the amount of peracid produced. The assay was carried out in 96-well microtiter plates and analyzed using a SpectroMax® Plus plate reader at 405 nm.

Whole cell paste was suspended in 0.1 M sodium acetate buffer (pH 4.0) at a final concentration of 50 mg/mL wet cell weight. A mixture of 100 µL of cell suspension, 30 µL of 0.1 M acetate buffer (pH 4.0), 20 µL of 313 mM hydrogen peroxide and 50 µL of 200 mM substrate were incubated at 37 °C for 15 minutes to 24 hours. Control samples were prepared by omitting hydrogen peroxide from the reaction mixture, and substituting an equivalent volume of deionized water. The reaction mixture was assayed by mixing 100 µL of the supernatant from a centrifuged reaction mixture with 50 µL of 1.5 M acetic acid containing 0.03 mg/mL potassium iodide and 50 µL of 1 mg/mL 2,2'-azino-bis-(3-ethylbenzothiazoline)-6-sulfonate (ABTS) in a microtiter plate well, incubating at room temperature for 10 minutes, and measuring the absorbance of the assay mixture at 405 nm.

### EXAMPLE 10

### Production of Peracetic Acid by Perhydrolysis of Triacetin with Hydrogen Peroxide Using E. coli Non-heme Haloperoxidase Transformants (pH 6.5)

Following the method for analysis of peracid production described in Example 8, the following *E. coli* transformants were assayed for perhydrolysis activity using triacetin and hydrogen peroxide. Due to the presence of catalase in these clones, it was necessary to add sodium azide (50 mM) as an inhibitor of catalase to the reaction mixture. Samples were analyzed approximately 24 hours after the reaction was initiated (Table 1). Controls for each transformant were performed by omitting the addition of H₂O₂, and all controls produced 0 ppm peracid.

**Table 1.**

| *E. coli* Cell/Plasmid ID | Peracetic acid in ppm using triacetin |
|---|---|
| TOP10/pSW167 (Ppu5B) | 3.8 |
| TOP10/pSW169 (Ppu5B) | 8.4 |
| TOP10/pSW166 (AtuA1) | 0.9 |
| TOP10/pSW173 (AtuA2) | 6.4 |
| TOP10/pSW 171 (AtuA2) | 7.2 |
| TOP10/pSW170 (AtuA3) | 0.8 |
| TOP10/pSW172 (AtuA4) | 1.2 |
| BL21/pSW174 (Ppu5B) | 4.6 |
| BL21/pSW175 (AtuA1) | 4.6 |
| BL21/pSW176 (AtuA2) | 10 |
| BL21/pSW177 (AtuA3) | 6 |
| BL21/pSW178 (AtuA4) | 4 |
| Control (no H₂O₂) | 0 |

### EXAMPLE 11

### Production of Peracetic Acid by Perhydrolysis of Ethyl Acetate with Hydrogen Peroxide Using E. coli Non-heme Haloperoxidase Transformants (pH 6.5)

Following the method for analysis of peracid production described in Example 8, the following *E. coli* transformants were assayed for perhydrolysis activity using ethyl acetate and hydrogen peroxide. Due to the presence of catalase in these clones, it was necessary to add sodium azide (50 mM) as an inhibitor of catalase to the reaction mixture. Samples were analyzed approximately 24 hours after the reaction was initiated (Table 2). Controls for each transformant were performed by omitting the addition of H₂O₂, and all controls produced 0 ppm peracid.

**Table 2.**

| *E. coli* Cell/Plasmid ID | Peracetic acid (ppm) |
|---|---|
| TOP10/pSW167 (Ppu5B) | 5.4 |
| TOP10/pSW169 (Ppu5B) | 0.8 |
| TOP10/pSW166 (AtuA1) | 0 |
| TOP10/pSW173 (AtuA2) | 0 |
| TOP10/pSW171 (AtuA2) | 0 |
| TOP10/pSW170 (AtuA3) | 1.0 |
| TOP10/pSW172 (AtuA4) | 0.8 |
| BL21/pSW174 (Ppu5B) | 5.6 |
| BL21/pSW175 (AtuA1) | 1 |
| BL21/pSW176 (AtuA2) | 0 |
| BL21/pSW177 (AtuA3) | 0 |
| BL21/pSW178 (AtuA4) | 2 |
| Control (no H₂O₂) | 0 |

### EXAMPLE 12

### Production of Peracetic Acid by Perhydrolysis of Triacetin with Hydrogen Peroxide Using E. coli Non-heme Haloperoxidase Transformants (pH 6.5)

Following the method for analysis of peracid production described in Example 8, the following *E. coli* transformants were assayed for perhydrolysis activity using triacetin and hydrogen peroxide. The *E. coli* host UM2 is catalase negative, and no catalase inhibitor was added to the reactions. Samples were analyzed approximately 30 min after the reaction was initiated (Table 3). Controls for each transformant were performed by omitting the addition of H₂O₂, and all controls produced 0 ppm peracid.

**Table 3.**

| *E. coli* Cell/Plasmid ID | Peracetic acid (ppm) |
|---|---|
| UM2/pSW166 (AtuA1) | 4.3 |
| UM2/pSW167 (Ppu5B) | 2.3 |
| Control (no H₂O₂) | 0 |

### EXAMPLE 13

### Production of Peracetic Acid by Perhydrolysis of Ethyl Acetate with Hydrogen Peroxide Using E. coli Non-heme Haloperoxidase Transformants (pH 6.5)

Following the method for analysis of peracid production described in Example 8, the following *E. coli* transformants were assayed for perhydrolysis activity using ethyl acetate and hydrogen peroxide. The *E. coli* host UM2 is catalase negative, and no catalase inhibitor was added to the reactions. Samples were analyzed approximately 30 min after the reaction was initiated (Table 4). Controls for each transformant were performed by omitting the addition of H₂O₂, and all controls produced 0 ppm peracid.

**Table 4.**

| *E. coli* Cell/Plasmid ID | Peracetic acid (ppm) |
|---|---|
| UM2/pSW166 (AtuA1) | 0.78 |
| UM2/pSW167 (Ppu5B) | 3.6 |
| Control (no H₂O₂) | 0 |

### EXAMPLE 14

### Production of Perlactic Acid by Perhydrolysis of Ethyl Lactate with Hydrogen Peroxide Using E. coli Non-heme Haloperoxidase Transformants (pH 6.5)

Following the method for analysis of peracid production described in Example 8, the following *E. coli* transformants were assayed for perhydrolysis activity using ethyl lactate and hydrogen peroxide. The *E. coli* host UM2 is catalase negative, and no catalase inhibitor was added to the reactions. Samples were analyzed approximately 30 min after the reaction was initiated (Table 5). Controls for each transformant were performed by omitting the addition of H₂O₂, and all controls produced 0 ppm peracid.

**Table 5**

| *E. coli* Cell/Plasmid ID | Perlactic acid (ppm) |
|---|---|
| UM2/pSW166 (AtuA1) | 0.78 |
| UM2/pSW167 (Ppu5B) | 3.0 |
| Control (no H₂O₂) | 0 |

### EXAMPLE 15

### Production of Peracetic Acid by Perhydrolysis of Triacetin with Hydrogen Peroxide Using E. coli Non-heme Haloperoxidase Transformants (pH 4.0)

Following the method for analysis of peracid production described in Example 9, the following *E. coli* transformants were assayed for perhydrolysis activity using triacetin and hydrogen peroxide. The *E. coli* host UM2 is catalase negative, and no catalase inhibitor was added to the reactions. Samples were analyzed approximately 30 min after the reaction was initiated (Table 6). Controls for each transformant were performed by omitting the addition of H₂O₂, and all controls produced 0 ppm peracid.

**Table 6.**

| *E. coli* Cell/Plasmid 10 | Peracetic acid (ppm) |
|---|---|
| UM2/pSW166 (AtuA1) | 1.6 |
| UM2/pSW167 (Ppu5B) | 0.75 |
| Control (no H₂O₂) | 0 |

### EXAMPLE 16

### Production of Peracetic Acid by Perhydrolysis of Ethyl Acetate with Hydrogen Peroxide Using E. coli Non-heme Haloperoxidase Transformants (pH 4.0)

Following the method for analysis of peracid production described in Example 9, the following *E. coli* transformants were assayed for perhydrolysis activity using ethyl acetate and hydrogen peroxide. The *E. coli* host UM2 is catalase negative, and no catalase inhibitor was added to the reactions. Samples were analyzed approximately 30 min after the reaction was initiated (Table 7). Controls for each transformant were performed by omitting the addition of H₂O₂, and all controls produced 0 ppm peracid.

**Table 7.**

| *E. coli* Cell/Plasmid ID | Peracetic acid (ppm) |
|---|---|
| UM2/pSW166 (AtuA1) | 1.3 |
| UM2/pSW167 (Ppu5B) | 0.66 |
| Control (no H₂O₂) | 0 |

### EXAMPLE 17

### Production of Perlactic Acid by Perhydrolysis of Ethyl Lactate with Hydrogen Peroxide Using E. coli Non-heme Haloperoxidase Transformants (pH 4.0)

Following the method for analysis of peracid production described in Example 9, the following *E. coli* transformants were assayed for perhydrolysis activity using ethyl lactate and hydrogen peroxide. The *E. coli* host UM2 is catalase negative, and no catalase inhibitor was added to the reactions. Samples were analyzed approximately 30 min after the reaction was initiated (Table 8). Controls for each transformant were performed by omitting the additional of H₂O₂, and all controls produced 0 ppm peracid.

**Table 8.**

| *E. coli* Cell/Plasmid ID | Perlactic acid (ppm) |
|---|---|
| UM2/pSW166 (AtuA1) | 1.5 |
| UM2/pSW167 (Ppu5B) | 0.68 |
| Control (no H₂O₂) | 0 |

### EXAMPLE 18

### Production of Peracetic Acid by Perhydrolysis of Triacetin with Hydrogen Peroxide Using Pseudomonas putida 5B Non-heme Haloperoxidase pH 6.5)

Following the method for analysis of peracid production described in Example 8, *P. putida* 5B was assayed for perhydrolysis activity using triacetin and hydrogen peroxide. Approximately 10 mM hydroxylamine sulfate was used as inhibitor of catalase activity in the reaction mixture. The sample was analyzed approximately 30 min after the reaction was initiated, and 3.7 ppm peracetic acid was produced.

### EXAMPLE 19

### Production of Peracetic Acid by Perhydrolysis of Ethyl Acetate with Hydrogen Peroxide Using Pseudomonas putida 5B Non-heme Haloperoxidase (pH 6.5)

Following the method for analysis of peracid production described in Example 8, P. *putida* 5B was assayed for perhydrolysis activity using ethyl acetate and hydrogen peroxide. Approximately 10 mM hydroxylamine sulfate was used as inhibitor of catalase activity in the reaction mixture. The sample was analyzed approximately 30 min after the reaction was initiated, and 2.0 ppm peracetic acid was produced.

### EXAMPLE 20

### Production of Perlactic Acid by Perhydrolysis of Ethyl Lactate with Hydrogen Peroxide Using Pseudomonas putida 5B Non-heme Haloperoxidases (pH 6.5)

Following the method for analysis of peracid production described in Example 8, *P. putida* 5B was assayed for perhydrolysis activity using ethyl lactate and hydrogen peroxide. Approximately 10 mM hydroxylamine sulfate was used as inhibitor of catalase activity in the reaction mixture. The sample was analyzed approximately 30 min after the reaction was initiated, and 3.2 ppm perlactic acid was produced

## Claims

1. A process for producing peracids from carboxylic acid ester substrates comprising
a) providing a set of peracid reaction components, said components comprising:
1) a carboxylic acid ester substrate selected from the group consisting of:
i) esters having the structure 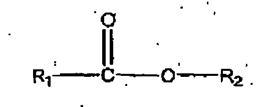 wherein R₁ = C1 to C10 straight chain of branched chain alkyl optionally substituted with an hydroxyl or a C1 to C4 alkoxy group and R₂= C1 to C10 straight chain or branched chain alkyl group, (CH₂CH₂-O)ₙH or (CH₂CH(CH₃)-O)ₙH and n=1 to 10, and
ii) glycerides having the structure 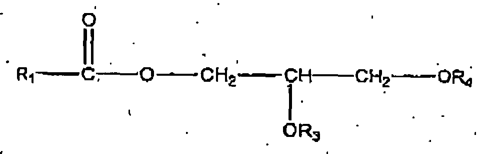 wherein R₁= C1 to C10 straight chain or branched chain alkyl optionally substituted with an hydroxyl or a C1 to C4 alkoxy group and R₃ and R₄ are individually H or R₁C(O); and
2) a source of peroxygen; and
3) a non-heme haloperoxidase catalyse having a perhydrolysis activity, wherein the non-heme haloperoxidase having perhydrolysis activity is encoded by an isolated nucleic acid molecule selected from the group consisting of:
a) an isolated nucleic acid molecule encoding a polypeptide having an amino acid sequence selected from the group consisting of SEQ ID NO: 10, SEQ ID NO: 14, SEQ ID Not, SEQ ID NO: 22, and SEQ ID NO: 28; and
b) an isolated nucleic acid molecule encoding a polypeptide having at least 95% identity to an amino acid sequence selected from the group consisting of SEQ ID NO: 10, SEQ ID NO: 14, SEQ ID NO: 18, SEQ ID NO: 22, and SEQ ID NO: 28;
and
b) combining said reaction components under aqueous reaction conditions, wherein said conditions comprising a pH range of about 2 to about 7.5, whereby a peracid is produced at a concentration of at least 500 ppb within about 5 minutes to about 2 hours of combining the reaction components.

2. The process of claim 1 wherein the pH is less than about 6.5.

3. The process of claim 2 wherein the pH range is less than about 4.5.

4. The process of claims 1-3 wherein the ester substrate is selected from the group consisting of methyl lactate, ethyl lactate, methyl glycolate, ethyl glycolate, methyl methoxyacetate, ethyl methoxyacetate, methyl 3-hydroxybutyrate, ethyl 3-hydroxybutyrate, and mixtures thereof.

5. The process of claims 1-3 wherein the glyceride is selected from the group consisting of monoacetin, diacetin, triacetin, monopropionin, glyceryl dipropionate, tripropionin, monobutyrin, glyceryl dibutyrate, tributyrin, and mixtures thereof.

6. The process of claim 4 wherein the peracid produced is selected from the group consisting of peracetic acid, perpropionic acid, perbutyric acid, perlactic acid, perglycolic acid, permethoxyacetic acid, per-β-hydroxybutyric acid, and mixtures thereof.

7. The process of claim 6 wherein the peracid produced is peracetic acid.

8. A process to reduce a microbial population on a hard surface or inanimate object using an enzymatically produced aqueous peracid composition, said process comprising:
a) providing a set of peracid reaction components, said components comprising:
1. substrate selected from the group consisting of:
i) esters having the structure 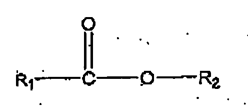 wherein R₁= C1 to C10 straight chain or branched chain and optionally , C10 substituted with an hydroxyl or a C1 to C4 alkoxy group and R₂= C1 to C10 straight chain or branched chain alkyl group, (CH₂CH₂-O)ₙH or (CH₂CH(CH₃)-O)ₙH and n=1 to 10; and
ii) glycerides having the structure 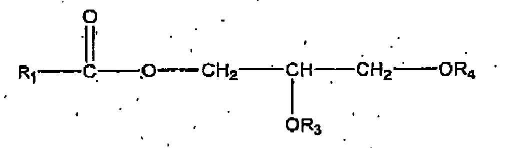 wherein R₁=C1 to C10 straight chain or branched chain alkyl optionally substituted with an hydroxyl or a C1 to C4 alkoxy group and R₃ and R₄ are individually H or R₁C(O); and
2) a source of peroxygen; and
3) a non-heme haloperoxidase catalyst having perhydrolysis activity, wherein the non-heme haloperoxidase having perhydrolysis activity is encoded by an isolated nucleic acid molecule selected from the group consisting of:
a) an isolated nucleic acid molecule encoding a polypeptide having an amino acid sequence selected from the group consisting of SEQ ID NO: 10, SEQ ID NO: 14, SEQ ID NO: 18, SEQ ID NO: 22, and SEQ ID NO: 28; and
b) an isolated nucleic acid molecule encoding a polypeptide having at least 95% identity to an amino acid sequence selected from the group consisting of SEQ ID NO: 10, SEQ ID NO: 14, SEQ ID NO: 18, SEQ ID NO: 22, and SEQ ID NO: 28;
b) providing a hard surface or an inanimate object having a concentration of microorganisms and/or viruses;
c) combining said reaction components under aqueous reaction conditions, wherein said conditions comprise a pH range of about 2 to about 7.5, whereby a peracid product is formed having a peracid concentration of at least 500 ppb within about 5 minutes to about 2 hour of combining the reaction components;
d) optionally diluting the said peracid producer and
e) contacting said hard surface or said inanimate object with the peracid produced in step c) or step d) within 48 hours of combining said reaction components thereby the concentration of said of micro-organisms is reduced at least 3-log.

9. The process of Claim 8 wherein the hard surface or the inanimate object is contacted with the peracid produced in step c) or step d) within about 30 minutes of combining said reaction components.

10. The process of Claim 9 wherein the hard surface or the inanimate object is contacted with the peracid produced in step c) or step d) within about 10 minutes of combining said reaction components.

11. The process according to Claims 8 to 10 wherein the concentration of said microorganisms is reduced at least 4-log.

12. The process of Claim 8 wherein the pH range is less than about 6.5.

13. The process of Claim 12 wherein the pH range is less than about 4.5.

14. The process of Claim 8 wherein the ester substrate is selected from the group consisting of methyl lactate, ethyl lactate, ethyl acetate, methyl glycolate, ethyl glycolate methyl methoxyacetate, ethyl methoxyacetate, methyl 3-hydroxybutyrate, ethyl 3-hydroxybutyrate, and mixtures thereof,

15. The process of claim 14 wherein the ester substrate is selected from the group consisting of ethyl lactate, ethyl acetate, and mixtures thereof.

16. The process of Claim 8 wherein the glyceride substrate is selected from the group consisting of monoacetin, diacetin, triacetin, monopropionin, glyceryl dipropionate, tripropionin, monobutyrin, glyceryl dibutyrate, tributyrin, and mixtures thereof.

17. The process of claim 16 wherein the glycercide substrate is selected from the group consisting of diacetin and triacetin.

18. An isolated nucleic acid molecule encoding a polypeptide having perhydrolytic activity wherein said polypeptide comprises an amino acid sequence having at least 98% identity to SEQ ID NO: 10.

19. The isolated nucleic acid molecule of claim 18 encoding a polypeptide having an amino acid sequence as represented by SEQ ID NO: 10.

20. The process of claim 8 , further comprising combining the peracid product of step (c), with one or more additional agents, including an antiseptic, disinfectant, virucide, biocide; antimicrobial agent, or mixtures thereof.

21. The process of claim 20, wherein the additional agent is a virucide selected from the group consisting of alcohols, ethers, chloroform, formaldehyde, phenols, beta propiolactone, iodine, chlorine, mercury salts, hydroxylamine, ethylene oxide, ethylene glycol, quaternary ammonium compounds, enzymes, and detergents.

22. The process of claim 20, wherein the Additional agent is a biocide selected from the group consisting of chlorine, chlorine dioxide, chloroisocyanurates, hypochlorites, ozone, acrolein, amines, chlorinated phenolics, copper salts, organo-sulphur compounds, and quaternary ammonium salts.

## Patentansprüche

1. Verfahren für die Herstellung von Persäuren aus Carbonsäureestersubstraten, umfassend
a) das Bereitstellen eines Satzes von Persäurereaktionskomponenten, wobei die Komponenten Folgendes umfassen:
1) ein Carbonsäureestersubstrat ausgewählt aus der Gruppe bestehend aus:
i) Estern, die die Struktur 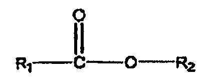 aufweisen, wobei R₁ = ein geradkettiges oder verzweigtes C₁- bis C₁₀-Alkyl, das wahlweise mit einer Hydroxy- oder einer C₁- bis C₄-Alkoxygruppe substituiert ist und R₂ = eine geradkettige oder verzweigtkettige C₁- bis C₁₀-Alkylgruppe, (CH₂CH₂-O)ₙH oder (CH₂CH(CH₃)-O)ₙH ist und n = 1 bis 10 beträgt; und
ii) Glyceriden, die die Struktur 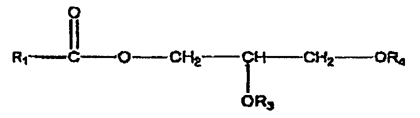 aufweisen, wobei R₁ = ein geradkettiges oder verzweigtes C₁-bis C₁₀-Alkyl ist, das wahlweise mit einer Hydroxy- oder einer C₁- bis C₄-Alkoxygruppe substituiert ist und R₃ und R₄ einzeln H oder R₁C(O) sind; und
2) eine Quelle von Persauerstoff; und
3) einen Nicht-Häm-Haloperoxidasekatalysator, der eine Perhydrolyseaktivität aufweist, wobei die Nicht-Häm-Haloperoxidase, die eine Perhydrolyseaktivität aufweist, durch ein isoliertes Nukleinsäuremolekül kodiert ist, ausgewählt aus der Gruppe bestehend aus:
a) einem isolierten Nukleinsäuremolekül, das ein Polypeptid kodiert, das eine Aminosäuresequenz aufweist, die aus der Gruppe ausgewählt ist bestehend aus SEQ ID NO: 10, SEQ ID NO: 14, SEQ ID NO: 18, SEQ ID NO: 22 und SEQ ID NO: 28; und
b) einem isolierten Nukleinsäuremolekül, das ein Polypeptid kodiert, das eine mindestens 95 %ige Identität mit einer Aminosäuresequenz aufweist, die aus der Gruppe ausgewählt ist bestehend aus SEQ ID NO: 10, SEQ ID NO: 14, SEQ ID NO: 18, SEQ ID NO: 22 und SEQ ID NO: 28;
und
b) das Kombinieren der Reaktionskomponenten unter wässrigen Reaktionsbedingungen, wobei die Bedingungen einen pH-Bereich von etwa 2 bis etwa 7,5 umfassen, wobei eine Persäure in einer Konzentration von mindestens 500 ppb innerhalb von etwa 5 Minuten bis etwa 2 Stunden nach Kombinieren der Reaktionskomponenten hergestellt wird.

2. Verfahren nach Anspruch 1, wobei der pH-Wert weniger als etwa 6,5 beträgt.

3. Verfahren nach Anspruch 2, wobei der pH-Wert weniger als etwa 4,5 beträgt.

4. Verfahren nach den Ansprüchen 1-3, wobei das Estersubstrat aus der Gruppe ausgewählt wird bestehend aus Methyllactat, Ethyllactat, Methylglykolat, Ethylglykolat, Methylmethoxyacetat, Ethylmethoxyacetat, Methyl-3-hydroxybutyrat, Ethyl-3-hydroxybutyrat und Mischungen derselben.

5. Verfahren nach den Ansprüchen 1-3, wobei das Glycerid aus der Gruppe ausgewählt wird bestehend aus Monoacetin, Diacetin, Triacetin, Monopropionin, Glyceryldipropionat, Tripropionin, Monobutyrin, Glyceryldibutyrat, Tributyrin und Mischungen derselben.

6. Verfahren nach Anspruch 4, wobei die hergestellte Persäure aus der Gruppe ausgewählt wird bestehend aus Peressigsäure, Perpropionsäure, Perbuttersäure, Permilchsäure, Perglykolsäure, Permethoxyessigsäure, Per-β-hydroxybuttersäure und Mischungen derselben.

7. Verfahren nach Anspruch 6, wobei die hergestellte Persäure Peressigsäure ist.

8. Verfahren zum Reduzieren einer mikrobiellen Population auf einer harten Oberfläche oder einem leblosen Gegenstand unter Anwendung einer enzymatisch hergestellten wässrigen Persäurezusammensetzung, wobei das Verfahren Folgendes umfasst:
a) das Bereitstellen eines Satzes von Persäurereaktionskomponenten, wobei die Komponenten Folgendes umfassen:
1) ein Substrat ausgewählt aus der Gruppe bestehend aus:
i) Estern, die die Struktur 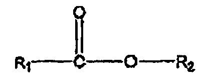 aufweisen, wobei R₁ = ein geradkettiges oder verzweigtes C₁- bis C₁₀-Alkyl, das wahlweise mit einer Hydroxy- oder einer C₁-bis C₄-Alkoxygruppe substituiert ist und R₂ = eine geradkettige oder verzweigtkettige C₁-bis C₁₀-Alkylgruppe, (CH₂CH₂-O)ₙH oder (CH₂CH(CH₃)-O)ₙH ist und n = 1 bis 10 beträgt; und
ii) Glyceriden, die die Struktur 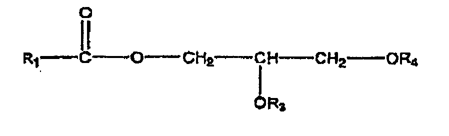 aufweisen, wobei R₁ = ein geradkettiges oder verzweigtes C₁- bis C₁₀-Alkyl ist, das wahlweise mit einer Hydroxy- oder einer C₁- bis C₄-Alkoxygruppe substituiert ist und R₃ und R₄ einzeln H oder R₁C(O) sind; und
2) eine Quelle von Persauerstoff; und
3) einen Nicht-Häm-Haloperoxidasekatalysator, der eine Perhydrolyseaktivität aufweist, wobei die Nicht-Häm-Haloperoxidase, die eine Perhydrolyseaktivität aufweist, durch ein isoliertes Nukleinsäuremolekül kodiert ist, ausgewählt aus der Gruppe bestehend aus:
a) einem isolierten Nukleinsäuremolekül, das ein Polypeptid kodiert, das eine Aminosäuresequenz aufweist, die aus der Gruppe ausgewählt ist bestehend aus SEQ ID NO: 10, SEQ ID NO: 14, SEQ ID NO: 18, SEQ ID NO: 22 und SEQ ID NO: 28; und
b) einem isolierten Nukleinsäuremolekül, das ein Polypeptid kodiert, das eine mindestens 95 %ige Identität mit einer Aminosäuresequenz aufweist, die aus der Gruppe ausgewählt ist bestehend aus SEQ ID NO: 10, SEQ ID NO: 14, SEQ ID NO: 18, SEQ ID NO: 22 und SEQ ID NO: 28;
b) das Bereitstellen einer harten Oberfläche oder eines leblosen Gegenstands, die/der eine Konzentration von Mikroorganismen und/oder Viren aufweist;
c) das Kombinieren der Reaktionskomponenten unter wässrigen Reaktionsbedingungen, wobei die Bedingungen einen pH-Bereich von etwa 2 bis etwa 7,5 umfassen, wobei ein Persäureprodukt, das eine Persäurekonzentration von mindestens 500 ppb aufweist, innerhalb von etwa 5 Minuten bis etwa 2 Stunden nach Kombinieren der Reaktionskomponenten gebildet wird;
d) das wahlweise Verdünnen des Persäureprodukts; und
e) das Kontaktieren der harten Oberfläche oder des leblosen Gegenstands mit der in Schritt c) oder Schritt d) hergestellten Persäure innerhalb von 48 Stunden nach Kombinieren der Reaktionskomponenten, wobei die Konzentration von Mikroorganismen um mindestens 3-log reduziert wird.

9. Verfahren nach Anspruch 8, wobei die harte Oberfläche oder der leblose Gegenstand mit der in Schritt c) oder Schritt d) hergestellten Persäure innerhalb von etwa 30 Minuten nach Kombinieren der Reaktionskomponenten in Kontakt gebracht wird.

10. Verfahren nach Anspruch 9, wobei die harte Oberfläche oder der leblose Gegenstand mit der in Schritt c) oder Schritt d) hergestellten Persäure innerhalb von etwa 10 Minuten nach Kombinieren der Reaktionskomponenten in Kontakt gebracht wird.

11. Verfahren nach den Ansprüchen 8 bis 10, wobei die Konzentration der Mikroorganismen um mindestens 4-log reduziert wird.

12. Verfahren nach Anspruch 8, wobei der pH-Wert weniger als etwa 6,5 beträgt.

13. Verfahren nach Anspruch 12, wobei der pH-Wert weniger als etwa 4,5 beträgt.

14. Verfahren nach Anspruch 8, wobei das Estersubstrat aus der Gruppe ausgewählt wird bestehend aus Methyllactat, Ethyllactat, Ethylacetat, Methylglykolat, Ethylglykolat, Methylmethoxyacetat, Ethylmethoxyacetat, Methyl-3-hydroxybutyrat, Ethyl-3-hydroxybutyrat und Mischungen derselben.

15. Verfahren nach Anspruch 14, wobei das Estersubstrat aus der Gruppe ausgewählt wird bestehend aus Ethyllactat, Ethylacetat und Mischungen derselben.

16. Verfahren nach Anspruch 8, wobei das Glyceridsubstrat aus der Gruppe ausgewählt wird bestehend aus Monoacetin, Diacetin, Triacetin, Monopropionin, Glyceryldipropionat, Tripropionin, Monobutyrin, Glyceryldibutyrat, Tributyrin und Mischungen derselben.

17. Verfahren nach Anspruch 16, wobei das Glyceridsubstrat aus der Gruppe ausgewählt wird bestehend aus Diacetin und Triacetin.

18. Isoliertes Nukleinsäuremolekül, das ein Polypeptid kodiert, das eine perhydrolytische Aktivität aufweist, wobei dsa Polypeptid eine Aminosäuresequenz umfasst, die eine mindestens 98 %-ige Identität mit SEQ ID NO: 10 aufweist.

19. Isoliertes Nukleinsäuremolekül nach Anspruch 18, das ein Polypeptid kodiert, das eine Aminosäuresequenz aufweist, wie sie durch SEQ ID NO: 10 dargestellt ist, aufweist.

20. Verfahren nach Anspruch 8, des Weiteren umfassend das Kombinieren des Persäureprodukts aus Schritt c) mit einem oder mehreren zusätzlichen Mittel(n), einschließlich eines Antiseptikums, Desinfektionsmittels, Viruzids, Biozids, antimikrobiellen Mittels oder von Mischungen derselben.

21. Verfahren nach Anspruch 20, wobei das zusätzliche Mittel ein Viruzid ist ausgewählt aus der Gruppe bestehend aus Alkoholen, Ethern, Chloroform, Formaldehyd, Phenolen, Beta-Propiolacton, Iod, Chlor, Quecksilbersalzen, Hydroxylamin, Ethylenoxid, Ethylenglykol, quartären Ammoniumverbindungen, Enzymen und Detergentien.

22. Verfahren nach Anspruch 20, wobei das zusätzliche Mittel ein Biozid ist ausgewählt aus der Gruppe bestehend aus Chlor, Chlordioxid, Chlorisocyanuraten, Hypochloriten, Ozon, Acrolein, Aminen, chlorierten Phenolen, Kupfersalzen, Organoschwefelverbindungen und quartären Ammoniumsalzen.

## Revendications

1. Procédé de fabrication de peracides à partir de substrats d'ester d'acide carboxylique, comprenant
a) la fourniture d'un ensemble de constituants de réaction peracides, lesdits constituants comprenant:
1) un substrat d'ester d'acide carboxylique choisi dans le groupe constitué par:
i) les esters ayant la structure 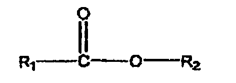 dans laquelle R₁= alkyle en C₁ à C₁₀ à chaîne linéaire ou à chaîne ramifiée, facultativement substitué par un hydroxyle ou un groupe alcoxy en C₁ à C₄ et R₂= groupe alkyle en C₁ à C₁₀ à chaîne linéaire ou à chaîne ramifiée, (CH₂CH₂-O)ₙH ou (CH₂CH(CH₃)-O)ₙH et n = 1 à 10; et
ii) les glycérides ayant la structure 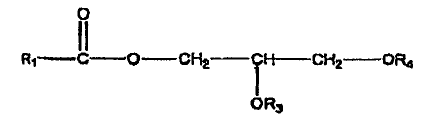 dans laquelle R₁= alkyle en C₁ à C₁₀ à chaîne linéaire ou à chaîne ramifiée, facultativement substitué par un hydroxyle ou un groupe alcoxy en C₁ à C₄ et R₃ et R₄ sont individuellement H ou R₁C(O); et
2) une source de peroxygène; et
3) un catalyseur halogénoperoxydase non hémique ayant une activité de perhydrolyse, dans lequel la halogénoperoxydase non hémique ayant une activité de perhydrolyse est codée par une molécule d'acide nucléique isolée choisie dans le groupe constitué par:
a) une molécule d'acide nucléique isolée codant un polypeptide ayant une séquence d'acides aminés choisie dans le groupe constitué par la SEQ ID NO: 10, la SEQ ID NO: 14, la SEQ ID NO: 18, la SEQ ID NO: 22 et la SEQ ID NO: 28; et
b) une molécule d'acide nucléique isolée codant un polypeptide ayant au moins 95% d'identité avec une séquence d'acides aminés choisie dans le groupe constitué par la SEQ ID NO: 10, la SEQ ID NO: 14, la SEQ ID NO: 18, la SEQ ID NO: 22 et la SEQ ID NO: 28;
et
b) la combinaison desdits constituants de réaction dans des conditions de réaction aqueuses, lesdites conditions comprenant une gamme de pH d'environ 2 à environ 7,5, de sorte qu'un peracide est produit à une concentration d'au moins 500 ppb en l'espace d'environ 5 minutes à environ 2 heures après la combinaison des constituants de réaction.

2. Procédé selon la revendication 1, dans lequel le pH est inférieur à environ 6,5.

3. Procédé selon la revendication 2, dans lequel la gamme de pH est inférieure à environ 4,5.

4. Procédé selon les revendications 1 à 3, dans lequel le substrat d'ester est choisi dans le groupe constitué par le lactate de méthyle, le lactate d'éthyle, le glycolate de méthyle, le glycolate d'éthyle, le méthoxyacétate de méthyle, le méthoxyacétate d'éthyle, le 3-hydroxybutyrate de méthyle, le 3-hydroxybutyrate d'éthyle, et des mélanges de ceux-ci.

5. Procédé selon les revendications 1 à 3, dans lequel le glycéride est choisi dans le groupe constitué par la monoacétine, la diacétine, la triacétine, la monopropionine, le dipropionate de glycéryle, la tripropionine, la monobutyrine, le dibutyrate de glycéryle, la tributyrine, et des mélanges de ceux-ci.

6. Procédé selon la revendication 4, dans lequel le peracide produit est choisi dans le groupe constitué par l'acide peracétique, l'acide perpropionique, l'acide perbutyrique, l'acide perlactique, l'acide perglycolique, l'acide perméthoxyacétique, l'acide per-β-hydroxybutyrique, et des mélanges de ceux-ci.

7. Procédé selon la revendication 6, dans lequel le peracide produit est l'acide peracétique.

8. Procédé pour réduire une population microbienne sur une surface dure ou un objet inanimé en utilisant une composition de peracide aqueuse produite enzymatiquement, ledit procédé comprenant:
a) la fourniture d'un ensemble de constituants de réaction peracides, lesdits constituants comprenant:
1) un substrat choisi dans le groupe constitué par:
i) les esters ayant la structure 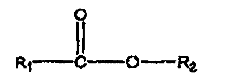 dans laquelle R₁= alkyle en C₁ à C₁₀ à chaîne linéaire ou à chaîne ramifiée, facultativement substitué par un hydroxyle ou un groupe alcoxy en C₁ à C₄ et R₂= groupe alkyle en C₁ à C₁₀ à chaîne linéaire ou à chaîne ramifiée, (CH₂CH₂-O)ₙH ou (CH₂CH(CH₃)-O)ₙH et n = 1 à 10; et
ii) les glycérides ayant la structure 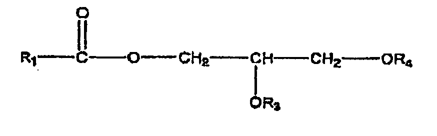 dans laquelle R₁= alkyle en C₁ à C₁₀ à chaîne linéaire ou à chaîne ramifiée, facultativement substitué par un hydroxyle ou un groupe alcoxy en C₁ à C₄ et R₃ et R₄ sont individuellement H ou R₁C(O); et
2) une source de peroxygène; et
3) un catalyseur halogénoperoxydase non hémique ayant une activité de perhydrolyse, dans lequel la halogénoperoxydase non hémique ayant une activité de perhydrolyse est codée par une molécule d'acide nucléique isolée choisie dans le groupe constitué par:
a) une molécule d'acide nucléique isolée codant un polypeptide ayant une séquence d'acides aminés choisie dans le groupe constitué par la SEQ ID NO: 10, la SEQ ID NO: 14, la SEQ ID NO: 18, la SEQ ID NO: 22 et la SEQ ID NO: 28; et
b) une molécule d'acide nucléique isolée codant un polypeptide ayant au moins 95% d'identité avec une séquence d'acides aminés choisie dans le groupe constitué par la SEQ ID NO: 10, la SEQ ID NO: 14, la SEQ ID NO: 18, la SEQ ID NO: 22 et la SEQ ID NO: 28;
b) la fourniture d'une surface dure ou d'un objet inanimé ayant une concentration de micro-organismes et/ou de virus;
c) la combinaison desdits constituants de réaction dans des conditions de réaction aqueuses, lesquelles conditions comprennent une gamme de pH d'environ 2 à environ 7,5, de sorte qu'il se forme un produit de peracide ayant une concentration de peracide d'au moins 500 ppb en l'espace d'environ 5 minutes à environ 2 heures après la combinaison des constituants de réaction;
d) facultativement, la dilution dudit produit de peracide; et
e) la mise en contact de ladite surface dure ou dudit objet inanimé avec le peracide produit dans l'étape c) ou l'étape d) en l'espace de 48 heures après la combinaison desdits constituants de réaction, de sorte que la concentration desdits micro-organismes est réduite d'au moins 3 logs.

9. Procédé selon la revendication 8, dans lequel la surface dure ou l'objet inanimé est mis(e) en contact avec le peracide produit dans l'étape c) ou l'étape d) en l'espace d'environ 30 minutes après la combinaison desdits constituants de réaction.

10. Procédé selon la revendication 9, dans lequel la surface dure ou l'objet inanimé est mis(e) en contact avec le peracide produit dans l'étape c) ou l'étape d) en l'espace d'environ 10 minutes après la combinaison desdits constituants de réaction.

11. Procédé selon les revendications 8 à 10, dans lequel la concentration desdits micro-organismes est réduite d'au moins 4 logs.

12. Procédé selon la revendication 8, dans lequel la gamme de pH est inférieure à environ 6,5.

13. Procédé selon la revendication 12, dans lequel la gamme de pH est inférieure à environ 4,5.

14. Procédé selon la revendication 8, dans lequel le substrat ester est choisi dans le groupe constitué par le lactate de méthyle, le lactate d'éthyle, l'acétate d'éthyle, le glycolate de méthyle, le glycolate d'éthyle, le méthoxyacétate de méthyle, le méthoxyacétate d'éthyle, le 3-hydroxybutyrate de méthyle, le 3-hydroxybutyrate d'éthyle, et des mélanges de ceux-ci.

15. Procédé selon la revendication 14, dans lequel le substrat ester est choisi dans le groupe constitué par le lactate d'éthyle, l'acétate d'éthyle, et des mélanges de ceux-ci.

16. Procédé selon la revendication 8, dans lequel le substrat glycéride est choisi dans le groupe constitué par la monoacétine, la diacétine, la triacétine, la monopropionine, le dipropionate de glycéryle, la tripropionine, la monobutyrine, le dibutyrate de glycéryle, la tributyrine, et des mélanges de ceux-ci.

17. Procédé selon la revendication 16, dans lequel le substrat glycéride est choisi dans le groupe constitué par la diacétine et la triacétine.

18. Molécule d'acide nucléique isolée codant un polypeptide ayant une activité perhydrolytique, lequel polypeptide comprenant une séquence d'acides aminés ayant au moins 98% d'identité avec la SEQ ID NO: 10.

19. Molécule d'acide nucléique isolée selon la revendication 18, codant un polypeptide ayant une séquence d'acides aminés représentée par la SEQ ID NO: 10.

20. Procédé selon la revendication 8, comprenant en outre la combinaison du produit de peracide de l'étape c) avec un ou plusieurs agents supplémentaires, incluant un antiseptique, un désinfectant, un virucide, un biocide, un agent antimicrobien, ou des mélanges de ceux-ci.

21. Procédé selon la revendication 20, dans lequel l'agent supplémentaire est un virucide choisi dans le groupe constitué par les alcools, les éthers, le chloroforme, le formaldéhyde, les phénols, la bêta propiolactone, l'iode, le chlore, les sels de mercure, l'hydroxylamine, l'oxyde d'éthylène, l'éthylèneglycol, les composés d'ammonium quaternaire, les enzymes et les détergents.

22. Procédé selon la revendication 20, dans lequel l'agent supplémentaire est un biocide choisi dans le groupe constitué par le chlore, le dioxyde de chlore, les chloroisocyanurates, les hypochlorites, l'ozone, l'acroléine, les amines, les substances phénoliques chlorées, les sels de cuivre, les composés organosoufrés et les sels d'ammonium quaternaire.
